# FASCICULE DE BREVET EUROPEEN

(11) **EP 3 377 608 B1**
(45) Date de publication et mention de la délivrance du brevet: **26.02.2020**
(21) Numéro de dépôt: 16798718.9
(22) Date de dépôt: 18.11.2016
(51) Int. Cl.: C12M 1/107, C12M 1/00

(54) **DISPOSITIF DE MÉTHANISATION À PARTIR DE BIOMASSE SOLIDE ET PROCÉDÉ DE PRODUCTION DE BIOGAZ CORRESPONDANT**
VORRICHTUNG ZUR METHANISIERUNG AUS FESTER BIOMASSE UND VERFAHREN ZUR HERSTELLUNG VON ENTSPRECHENDEM BIOGAS
DEVICE FOR METHANIZATION FROM SOLID BIOMASS AND PROCESS FOR PRODUCING CORRESPONDING BIOGAS

(30) Priorité: 20.11.2015 FR 1561206
(43) Date de publication de la demande: 26.09.2018
(73) Titulaire: YANNCO, 92800 Puteaux (FR)
(72) Inventeur: MERCIER, Yann, 92800 Puteaux (FR)
(74) Mandataire: Casalonga
(86) Numéro de dépôt international: PCT/EP2016/078196
(87) Numéro de publication internationale: WO 2017/085290

(56) Documents cités:
- EP-A1- 1 428 868
- EP-A1- 2 251 408
- EP-A1- 2 428 558
- CN-U- 202 482 313
- GB-A- 2 333 771

## Description

La présente invention concerne un digesteur de méthanisation destiné à la production de biogaz comprenant, au niveau du toit du digesteur, un espace d'introduction et d'évacuation d'un container contenant de la biomasse solide.

L'invention concerne également un procédé de production de biogaz ainsi qu'une installation de méthanisation mettant en œuvre une unité de digestion principale destinée notamment à alimenter en digestat liquide plusieurs digesteurs de méthanisation contenant de la biomasse solide.

Au sens de la présente invention, on entend par biomasse solide des matières organiques d'origine végétales, animales, d'origine urbaine, ou agricole, ou industrielle, ainsi que des matières bactériologiques ou fongiques. En particulier, les déchets urbains organiques solides correspondent notamment à des déchets organiques domestiques, des déchets organiques de restauration, des déchets organiques de cantine, des déchets organiques de la grande distribution. Les biomasses solides ont un taux moyen de matière sèche supérieur à 12%, de préférence supérieur à 15%. Elles sont biodégradables et permettent la production de biogaz.

La biomasse solide d'origine urbaine est généralement hétérogène et contient de nombreux éléments indésirables, issus notamment des déchets non organiques des ménages, tels que des sacs ou des morceaux de plastique, des restes d'emballage, des boites de conserve et autres objets métalliques, des objets en verre ou des morceaux de verre, des morceaux de tissu, des ficelles, et d'une façon générale tous types d'objets que l'on peut jeter par inadvertance dans une poubelle réservée aux déchets organiques, ou qui ont pu échapper aux processus de séparation mis en œuvres dans les usines de tri mécano-bio logique.

La biomasse solide d'origine agricole peut être fibreuse, notamment pailleuse, et/ou hétérogène, et/ou contenir des éléments indésirables, notamment des cailloux, du gravier, du sable, des barbelés, des ficelles, des pièces métalliques provenant d'outils agricoles ou encore des sacs en plastiques, des éléments d'emballage et de conditionnement.

La méthanisation (encore appelée digestion anaérobie) est un procédé biologique naturel qui met en œuvre la dégradation des matières organiques par plusieurs types de micro-organismes, en particulier des bactéries, dans des conditions contrôlées et ceci en l'absence d'oxygène.

Un tel procédé conduit, d'une part, à la production de biogaz, qui correspond à un mélange gazeux composé majoritairement de méthane et de dioxyde de carbone, et, d'autre part, à la production d'un produit humide riche en matières organiques partiellement stabilisées appelé digestat, qui peut généralement être ensuite utilisé comme amendement organique.

Le biogaz présente l'avantage d'être un gaz convertible en énergie renouvelable et peut par exemple servir dans la production d'électricité et/ou de chaleur ou dans la production de carburant. Le biogaz peut également être injecté dans un réseau de gaz naturel après épuration.

Le digestat peut subir un traitement de séparation de phase liquide/solide dont la fraction solide (appelé digestat solide) est plus riche en matières organiques et en éléments phosphatés et la fraction liquide (appelé digestat liquide) est plus riche en azote ammoniacal et en potassium. Les digestats liquides et solides peuvent être stockés, et/ou traités et/ou épandus séparément.

Ainsi le procédé biologique de méthanisation permet de valoriser des matières organiques, en produisant une énergie renouvelable, et de diminuer les émissions de gaz à effet de serre, en captant le méthane issu de la dégradation naturelle de ces matières, et en substituant l'emploi d'énergies fossiles et/ou d'engrais chimiques.

Les matières organiques, susceptibles de produire du biogaz par le biais d'une méthanisation, peuvent être des déchets d'origine urbaine ou bien provenir par exemple du secteur agricole et/ou agro-industriel. En particulier, les déchets de l'alimentation humaine issus des ménages, des restaurants, des cantines, ou de la grande distribution, ou encore le fumier, provenant de l'exploitation agricole d'élevages, est une matière organique, issue des déjections animales mélangées à la paille des litières, constituent des sources majeures et particulièrement intéressantes dans la production de biogaz.

La méthanisation est couramment mise en œuvre à l'intérieur d'un réacteur appelé digesteur correspondant le plus souvent à une cuve, ayant une forme le plus souvent essentiellement cylindrique, mais parfois parallélépipédique, verticale ou horizontale, qui est destinée à contenir les matières organiques à traiter, en particulier des déchets organiques d'origine urbaine, et/ou du fumier et/ou des pailles et/ou des déchets d'origine industrielle, afin de produire du biogaz et du digestat.

Les différents procédés de méthanisation peuvent être répartis en deux grandes familles en fonction de la nature des déchets à dégrader, à savoir les procédés de méthanisation en voie liquide et les procédés de méthanisation en voie sèche.

En premier lieu, le procédé de méthanisation en voie liquide est généralement mis en œuvre pour traiter des déchets dont le mélange a un taux moyen de matière sèche inférieur à 15%, correspondant par exemple à des mélanges de boues liquides ou de graisses ou des mélanges principalement composés de lisier. Un tel procédé peut être aussi utilisé pour traiter des matières organiques solides qui ont été mélangées avec de l'eau ou du digestat liquide.

Le procédé de méthanisation en voie liquide consiste notamment à acheminer de manière généralement continue les matières organiques à traiter, le plus souvent au moyen de systèmes de pompage ou de vis sans fin à l'intérieur d'un digesteur qui est généralement hermétiquement fermé par l'intermédiaire d'un toit ou d'une membrane souple. Les matières organiques à traiter sont continuellement brassées à l'intérieur du digesteur, souvent par un ou plusieurs agitateurs, pouvant être à hélices ou à pâles, ou parfois par des injections de gaz, notamment de biogaz, afin d'éviter les phénomènes de décantation de la biomasse au fond du digesteur et/ou les phénomènes de flottation et de croûtage de la biomasse en surface du liquide dans le digesteur, et de favoriser la formation de biogaz et le contact avec les micro-organismes. Le digesteur peut éventuellement être chauffé dans des températures comprises entre 20 et 60°C, en particulier dans des températures comprises entre 35 et 55°C. Les matières organiques séjournent en moyenne plusieurs semaines dans le digesteur, typiquement pendant une période pouvant aller de 15 à 80 jours.

Le biogaz, produit au cours de cette réaction de méthanisation, est généralement stocké à pression atmosphérique dans une membrane souple étanche fixée au-dessus du digesteur. La membrane se présente alors sous la forme d'un dôme contenant un ciel gazeux au-dessus de la paroi verticale du digesteur. Plus rarement, le biogaz peut être stocké dans un gazomètre, souvent une poche de stockage souple généralement située sur le sol à côté du digesteur.

Ce procédé de méthanisation en voie liquide présente notamment l'inconvénient que les déchets à dégrader peuvent être difficiles à manipuler et à préparer pour leur acheminement vers le digesteur et nécessitent une dépense d'énergie importante pour être brassés de manière continue à l'intérieur du digesteur.

Dans les procédés de méthanisation en voie liquide qui incluent l'introduction de matières organiques solides, les matières organiques solides sont souvent broyées avant d'être incorporées dans la cuve de digestion afin de faciliter l'acheminement des matières organiques au sein du digesteur ainsi que leur brassage. Parfois, notamment pour les déchets d'origine urbaine, les matières organiques solides font l'objet d'un processus de tri préalable afin d'en retirer le plus possible d'objets indésirables.

Ce type de procédé de méthanisation en voie liquide continue présente souvent des difficultés au niveau de l'alimentation de la biomasse car il est souvent nécessaire de traiter préalablement les déchets avant leur introduction dans le digesteur, notamment en broyant la partie solide de la biomasse et en extrayant les matières indésirables. De plus, le système de brassage au sein du digesteur peut être endommagé du fait de la nature des matières organiques ou des matières indésirables.

En second lieu, le procédé de méthanisation, dit par voie sèche, est mis en œuvre pour traiter des déchets solides dont le mélange a un taux moyen de matière sèche généralement supérieur à 15%, notamment allant de 15% à 60%, correspondant par exemple à des déchets urbains, du fumier, de la paille, des déchets industriels, ou des biomasses hétérogènes.

Un premier type de procédé de méthanisation par voie sèche consiste à acheminer en continu, notamment au moyen d'une trémie et d'un système de vis sans fin, ou plus rarement d'un système de pompage puissant, les matières organiques solides à traiter à l'intérieur d'un digesteur, le plus souvent disposé de manière horizontale. Les matières organiques solides à traiter sont brassées, le plus souvent lentement, à l'intérieur du digesteur qui peut être également chauffé dans des températures comprises entre 20 et 60°C, en particulier dans des températures comprises entre 35 et 55°C.

De plus, les matières organiques solides sont souvent broyées avant d'être incorporées dans la cuve de digestion afin de faciliter l'acheminement des matières organiques au sein du digesteur ainsi que leur brassage. Parfois, notamment pour les déchets d'origine urbaine, les matières organiques solides font aussi l'objet d'un processus de tri préalable afin d'en retirer le plus possible d'objets indésirables.
igestion constituée d'une percolation du digestat liquide à résente souvent des difficultés au niveau de l'alimentation de la biomasse car il est souvent nécessaire de traiter préalablement les déchets avant leur introduction dans le digesteur, notamment en les broyant et en extrayant les matières indésirables. De plus, le système de brassage au sein du digesteur peut être endommagé du fait de la nature des matières organiques ou des matières indésirables.

Un second type de procédé de méthanisation en voie sèche est un procédé discontinu, ou en bâchées. Il consiste à acheminer, souvent au moyens d'engins mécaniques de chargement/déchargement, par exemple de type chargeur à godets, les matières organiques solides à l'intérieur de cellules de méthanisation qui sont fermées une fois remplies. Ces cellules sont généralement des sortes de garages équipés de portes que l'on referme une fois le chargement réalisé. Les matières organiques sont ensuite aspergées ou douchées avec du digestat liquide, chargé en bactéries, afin de réaliser une percolation conduisant à la production de biogaz. Une fois la réaction terminée, les portes de chaque cellule sont ouvertes afin de récupérer le digestat solide, généralement au moyen des mêmes engins mécaniques que pour le chargement. Un tel procédé met généralement en œuvre plusieurs cellules de méthanisation ou digesteurs, alimentées en bâchées l'une après l'autre. Dans ce procédé, les cellules sont généralement destinées à fonctionner majoritairement en même temps même si elles ont été alimentées à des moments différents. Le biogaz, produit au cours de la réaction de méthanisation, est le plus souvent stocké séparément dans un gazomètre, souvent une poche de stockage souple située à côté des cellules de méthanisation.

Ce type de procédé de méthanisation en voie sèche discontinue pose notamment le problème de la gestion du biogaz, susceptible d'être rejeté dans l'atmosphère pendant les périodes de remplissage et de vidange des cellules de méthanisation. De plus, la réaction de méthanisation n'est pas convenablement optimisée car la biomasse, introduite dans ces cellules, n'est pas mélangée et souvent des chemins préférentiels de circulation du digestat liquide dans la biomasse solide se forment, empêchant une bonne diffusion du digestat liquide au sein la biomasse solide à traiter, et donc empêchant un contact intime entre les bactéries et la biomasse, réduisant sensiblement le taux de dégradation de la biomasse solide.

Ainsi les matières organiques à traiter soulèvent souvent des difficultés au cours des différents procédés de méthanisation car ils présentent l'inconvénient de comporter de nombreux éléments indésirables et/ou d'être difficiles à manipuler et/ou à broyer.

En effet, les éléments indésirables sont souvent difficiles à séparer de la biomasse à traiter et peuvent donc endommager de manière importante les installations composant l'unité de méthanisation. A titre d'exemple, ces éléments indésirables peuvent endommager les pompes, les vis sans fin, les broyeurs ou les agitateurs conduisant ainsi au remplacement partiel ou total des pièces d'usure et/ou des pièces principales selon des fréquences élevées, engendrant des coûts de maintenance élevés.

De tels dégâts sont donc susceptibles d'entraîner une diminution dans la production de biogaz, voire un arrêt de celle-ci, et d'induire des manques à gagner et/ou une augmentation des coûts de maintenance et d'exploitation de l'installation.

De plus, de nombreux types de biomasses solides sont très difficiles à brasser dans le digesteur car ils ont tendance à flotter en surface et à s'emmêler, ce qui conduit à des phénomènes d'accumulation et de croûtage qui empêchent notamment la bonne circulation du biogaz au sein de la cuve, compliquant ainsi sa récupération, et qui peut aboutir à la prise en masse complète de la biomasse solide présente dans le digesteur et la paralysie de celui-ci.

A l'inverse, d'autres déchets, en particulier les éléments indésirables des déchets urbains, ou encore le sable ou les cailloux contenus dans certains déchets agricoles, ont tendance à décanter au fond du digesteur et à s'y accumuler, ce qui peut aboutir à la nécessité d'arrêter le digesteur et de le vider pour procéder à son nettoyage, ce qui peut faire perdre plusieurs semaines de production à l'unité de méthanisation. GB2333771 divulgue un système de méthanisation pour le traitement des résidus solides et génération de biogaz. Le système comporte un digesteur de méthanisation, destiné à la production de biogaz, comportant une cuve apte à se remplir de digestat liquide et à contenir un container contenant de la biomasse solide.

Ainsi les différentes unités de méthanisation mises en œuvre à l'heure actuelle présentent un certain nombre d'inconvénients quand elles doivent traiter en totalité ou en partie des biomasses solides.

Au vu de ce qui précède, l'invention a notamment pour but de valoriser plus efficacement la biomasse solide tout en minimisant les dépenses d'énergie, en réduisant les dépenses de personnel d'exploitation, en rendant non nécessaire l'achat et l'installation de certains équipements de chargement/déchargement, de broyage, de préparation et d'agitation de la biomasse solide, en diminuant les risques d'endommagement des installations composant l'unité de méthanisation et en diminuant les coûts de maintenance et de préparation de la biomasse solide.

La présente invention a notamment pour objet un digesteur de méthanisation, destiné à la production de biogaz, comportant une cuve, apte à contenir un container contenant de la biomasse solide, ledit container étant apte à se remplir de digestat liquide et ledit digesteur comportant au niveau de sa partie supérieure :
- au moins une zone d'introduction du container contenant la biomasse solide et d'évacuation du container contenant de la biomasse solide digérée résiduelle ; zone sur laquelle un toit est apte à être ouvert et refermé, et
- au moins une zone d'évacuation du biogaz.

De préférence, la zone d'admission et d'évacuation du container et la zone d'évacuation du biogaz sont séparées l'une de l'autre.

La zone d'admission, située en particulier sur au moins une partie de la surface supérieure de la cuve sur laquelle le toit peut être ouvert et refermé, permet l'incorporation d'un container contenant de la biomasse solide à l'intérieur de la cuve et l'évacuation de ce container contenant de la biomasse solide digérée une fois la digestion terminée, c'est-à-dire contenant du digestat solide (encore appelé biomasse solide digérée résiduelle). En d'autres termes, la zone d'admission est configurée de manière à pouvoir recevoir un container contenant de la biomasse solide et à évacuer, une fois la digestion terminée, un tel container contenant de la biomasse résiduelle digérée.

La zone d'admission permet donc d'introduire directement le container contenant la biomasse solide dans le digesteur sans avoir à effectuer préalablement d'opération de déchargement de la biomasse contenue dans ce container, ni d'opération de séparation des éléments indésirables contenus dans la biomasse solide ou de dilution préalable de la biomasse solide dans un liquide ou un broyage, ce qui permet de diminuer les investissements dans les équipements de déchargement, de préparation de la biomasse solide, de diminuer les dépenses énergétiques, de maintenance et de personnel et les risques d'endommagement par rapport à des unités de méthanisation classiques.

Ainsi le digesteur selon l'invention comporte au niveau de sa partie supérieure une zone configurée à la fois pour recevoir un container contenant de la biomasse solide et évacuer, après digestion, le container contenant de la biomasse solide digérée résiduelle.

En particulier, le digesteur selon l'invention permet de s'affranchir d'un dispositif visant à séparer les éléments indésirables de la biomasse solide, d'un dispositif de broyage et d'un dispositif d'agitation de la biomasse dans le digesteur.

Le container selon l'invention est apte à se remplir de digestat liquide lorsqu'il est immergé dans le digestat liquide.

Le container selon l'invention est préférentiellement conçu de telle sorte qu'il soit étanche lorsqu'il se trouve en dehors du digesteur, mais que, une fois introduit dans la cuve du digesteur, le digestat liquide puisse traverser verticalement ledit container sans que de la biomasse solide puisse s'échapper dudit container ; sauf éventuellement de façon très limitée.

Après introduction du container contenant la biomasse solide, et fermeture du toit, la cuve du digesteur est remplie avec du digestat liquide, qui est acheminé par l'intermédiaire d'une ou plusieurs pompes d'alimentation, en provenance d'une unité de digestion apte à stocker du digestat liquide et du biogaz (appelée unité de digestion principale et qui peut être composée d'un ou plusieurs ouvrages). Après remplissage de la cuve par du digestat liquide, la méthanisation se déroule par percolation du digestat liquide à travers le container, donc à travers la biomasse solide et diffusion du digestat liquide dans la biomasse solide, laquelle est totalement immergée (et éventuellement partiellement mise en suspension), dans des conditions anaérobies au sein du container situé dans la cuve du digesteur.

La digestion de la biomasse solide par percolation du digestat liquide à travers la biomasse solide et diffusion du digestat liquide dans la biomasse solide permet de s'affranchir de la nécessité d'agiter le mélange de biomasse solide et de biomasse liquide dans la cuve du digesteur, évitant ainsi des investissements en moyens mécaniques d'agitation et des coûts d'exploitation, notamment en consommation électrique.

La zone d'évacuation du biogaz, située au niveau de la partie supérieure du digesteur, permet de récupérer un mélange de biogaz et de digestat liquide qui va ensuite être acheminé vers l'unité de digestion principale apte à stocker du biogaz et du digestat liquide.

De préférence, la zone d'évacuation du biogaz est localisée au niveau de la surface supérieure de la cuve du digesteur.

Une fois la méthanisation terminée, une vidange aérobie du digestat liquide contenu dans la cuve du digesteur est réalisée, au cours de laquelle l'espace vacant laissé par cette vidange est rempli par de l'air, et l'accès à travers la zone d'admission permet alors de récupérer facilement le container contenant la biomasse solide digérée résiduelle, une fois la digestion terminée, en minimisant l'énergie dépensée au cours de cette opération. Il s'agit donc d'un procédé de méthanisation discontinu, ou en bâchées.

En d'autres termes, la zone d'admission constitue un orifice d'entrée et de sortie d'un container contenant de la biomasse solide, orifice situé au niveau de la surface supérieure de la cuve du digesteur selon l'invention.

Le digesteur selon l'invention est relié, via un circuit d'alimentation en digestat liquide et un circuit d'évacuation du biogaz et du digestat liquide, à une unité de digestion principale apte à contenir du biogaz et du digestat liquide. De cette façon, une circulation permanente, en aller et retour, du digestat liquide peut être mise en œuvre entre le digesteur et l'unité de digestion principale.

Le digesteur selon l'invention permet donc de valoriser plus efficacement la biomasse solide en réduisant les investissements en divers équipements, les dépenses énergétiques, de personnel et de maintenance des équipements, l'usure des matériels et les risques d'endommagement.

Selon une caractéristique de l'invention, le digesteur est surmonté d'un système d'accrochage apte à permettre l'introduction du container contenant la biomasse solide à l'intérieur de la cuve et l'évacuation du container contenant de la biomasse solide digérée résiduelle.

En d'autres termes, le système d'accrochage est apte à introduire le container contenant la biomasse solide dans le digesteur et à décharger le container contenant la biomasse solide digérée résiduelle, une fois la digestion terminée, à travers la zone d'admission du digesteur.

Le système d'accrochage a pour fonction de saisir le container contenant la biomasse solide et de l'introduire dans le digesteur sans avoir à effectuer une étape de déchargement de la biomasse contenue dans le container, de tri ou de séparation préalable des éléments indésirables susceptibles d'endommager les installations composant l'unité de méthanisation, et sans avoir à effectuer une étape de broyage ni de dilution de la biomasse solide.

Le système d'accrochage a également pour fonction de récupérer le container contenant la biomasse solide digérée résiduelle une fois la digestion terminée, c'est-à-dire contenant le digestat solide, et d'évacuer le container du digesteur.

Ainsi le système d'accrochage permet d'alimenter et de décharger le digesteur par le haut à travers la zone d'admission (respectivement d'évacuation) située sur au moins une partie de la surface supérieure de la cuve sur laquelle se referme le toit.

Le système d'accrochage peut également avoir pour fonction d'ouvrir le toit du digesteur avant d'alimenter le digesteur en biomasse solide ou de décharger le container contenant le digestat solide.

Le système d'accrochage peut également servir à fermer le toit du digesteur après les opérations d'introduction dans le digesteur du container contenant de la biomasse solide ou de déchargement du container contenant du digestat solide.

Le système d'accrochage présente donc l'avantage de pouvoir acheminer le container jusqu'au digesteur, de l'introduire dans la cuve du digesteur, et de l'évacuer, une fois la digestion terminée, vers une aire de stockage ou d'évacuation.

Le système d'accrochage est préférentiellement constitué de un ou plusieurs crochets aptes à saisir le container contenant la biomasse solide, de préférence trois crochets, lequel container est équipé d'une ou de plusieurs anses qui permettent de le saisir en y accrochant le ou les crochets du système d'accrochage. De préférence, le système d'accrochage est équipé de trois crochets, tandis que le container est équipé de trois anses, afin d'assurer une parfaite verticalité du container lors de son acheminement et son introduction dans le digesteur, et lors de son évacuation du digesteur.

La zone d'évacuation de biogaz, située au niveau de la partie supérieure du digesteur permet d'évacuer le biogaz et également du digestat liquide par débordement.

En particulier, l'évacuation du biogaz peut se faire et se fait de préférence concomitamment à la recirculation du digestat liquide vers l'unité de digestion principale.

Selon une autre caractéristique de l'invention, le digesteur comprend au moins un élément de séparation perforé inférieur, situé au niveau de la partie inférieure du digesteur, apte à séparer le digestat liquide et la biomasse solide, en particulier la biomasse qui se serait éventuellement échappée du container pendant les opérations de chargement/déchargement ou durant la phase de digestion, laquelle séparation est avantageuse notamment lors des vidanges aérobies et anaérobies du digestat liquide vers l'unité de digestion principale.

De préférence, les éléments de séparation perforés inférieur, situés au niveau de la partie inférieure de la cuve du digesteur, se trouvent en amont du système de vidange et d'évacuation du digestat liquide vers l'unité de digestion principale.

De préférence, l'élément de séparation perforé inférieur, situé au niveau de la partie inférieure du digesteur, est un tuyau perforé dont l'extrémité est obturée partiellement ou totalement.

Selon une caractéristique de l'invention, le digesteur comprend une ouverture munie d'une vanne apte à obturer ou permettre le passage de l'air entre l'intérieur et l'extérieur de la cuve du digesteur et une vanne destinée à obturer ou permettre la circulation du biogaz et du digestat liquide entre le digesteur et l'unité de digestion principale.

La vanne, apte à obturer ou permettre le passage de l'air entre l'intérieur et l'extérieur de la cuve du digesteur, permet d'introduire de l'air à l'intérieur du digesteur, notamment au moment de la mise en œuvre d'une étape de vidange aérobie, et permet d'évacuer l'air au moment du remplissage du digesteur avec du digestat liquide après l'introduction dans le digesteur du container contenant de la biomasse solide.

La vanne, apte à obturer ou permettre la circulation du biogaz et du digestat liquide, permet de les faire circuler dans les circuits d'acheminement reliés à l'unité de digestion principale.

Selon une caractéristique de l'invention, le digesteur comprend un compresseur d'air apte à injecter de l'air dans la partie inférieure de la cuve du digesteur.

Le compresseur d'air a pour fonction d'introduire de l'air à l'intérieur du digesteur, dans la partie inférieure de la cuve du digesteur, notamment au moment de la mise en œuvre d'une étape de vidange aérobie, afin de purger l'intérieur du container des éventuelles bulles de biogaz qui pourraient y être restées piégées dans la biomasse digérée, et afin de favoriser le passage du digestat liquide à travers la biomasse et son évacuation par le bas grâce aux remous que les bulles d'air provoque dans la biomasse contenue dans le container.

Selon une caractéristique de l'invention, le digesteur comprend un compresseur de biogaz, en provenance d'une unité de digestion principale, apte à injecter du biogaz dans la partie inférieure de la cuve du digesteur.

Le compresseur de biogaz a pour fonction d'introduire du biogaz en provenance de l'unité de digestion principale à l'intérieur du digesteur, dans la partie inférieure de la cuve du digesteur, notamment au moment de la mise en œuvre d'une étape de vidange anaérobie, afin de favoriser le passage du digestat liquide à travers la biomasse et l'évacuation par la partie d'inférieure du digestat liquide grâce aux remous que les bulles d'air provoque dans la biomasse contenue dans le container.

L'injection de biogaz en provenance de l'unité de digestion principale à l'intérieur du digesteur, dans la partie inférieure de la cuve du digesteur, peut également se faire en dehors des périodes de vidange anaérobie du digesteur, pendant les périodes de digestion anaérobie, afin d'agiter la biomasse solide et ainsi casser les éventuels chemins préférentiels de circulation du digestat liquide à travers la biomasse solide et favoriser le contact intime entre les bactéries anaérobies et la biomasse solide.

L'unité de digestion principale peut correspondre à un digesteur apte à stocker conjointement du digestat liquide et du biogaz ou à deux cuves distinctes capables de stocker séparément du digestat liquide et du biogaz. Dans certains cas, l'unité de digestion principale peut comporter plusieurs digesteurs aptes à stocker conjointement du digestat liquide et du biogaz ou plusieurs cuves distinctes capables de stocker séparément du digestat liquide et du biogaz.

Selon une caractéristique de l'invention, le toit apte à s'ouvrir et se fermer situé sur la partie supérieure du digesteur peut être surmonté d'un bouchon permettant d'assurer une meilleure étanchéité du toit et d'éviter les fuites éventuelles de biogaz. Dans certains cas, ce bouchon peut être un bouchon liquide, le liquide pouvant notamment être de l'eau ou du digestat liquide.

L'invention a également pour objet un procédé de production de biogaz comprenant successivement :
- une étape d'introduction dans le digesteur du container contenant la biomasse solide, mise en œuvre par au moins un système d'accrochage, à travers une zone d'admission et d'évacuation située au niveau de la partie supérieure d'une cuve d'un digesteur selon l'invention et sur laquelle un toit est apte à s'ouvrir et à se refermer,
- une étape de remplissage de la cuve du digesteur contenant le container avec un digestat liquide pour immerger la biomasse solide contenue dans le container et éventuellement mettre en suspension une partie de ladite biomasse solide dans le container,
- une étape de digestion constituée d'une percolation du digestat liquide à travers la biomasse contenue dans le container, et d'une diffusion du digestat liquide dans la biomasse solide, afin de mettre en contact intime les bactéries contenues dans le digestat liquide et la biomasse solide, dans des conditions anaérobies, pour générer puis récupérer le biogaz à travers une zone d'évacuation située dans la partie supérieure du digesteur,
- une étape de vidange aérobie du digestat liquide de la cuve du digesteur, et
- une étape d'évacuation du container contenant de la biomasse solide digérée résiduelle, mise en œuvre par au moins un système d'accrochage, à travers la zone d'évacuation du digesteur.

En d'autres termes, la cuve du digesteur est alimentée par le haut avec un container contenant de la biomasse solide à travers une zone sur laquelle le toit du digesteur s'ouvre et se referme.

De préférence, l'étape de vidange aérobie consiste en un pompage à partir de la partie inférieure de la cuve du digesteur vers l'unité de digestion principale à travers l'élément de séparation inférieur. Durant cette étape, l'espace vacant laissé par l'évacuation du digestat liquide est rempli par de l'air en provenance de l'extérieur de la cuve.

Durant cette étape, de l'air peut être injecté dans le digesteur, dans la partie inférieure de la cuve du digesteur, au moyen d'un compresseur d'air, afin de purger l'intérieur du container des éventuelles bulles de biogaz qui pourraient être y restées piégées dans la biomasse digérée, et afin de favoriser le passage du digestat liquide à travers la biomasse et son évacuation par le bas grâce aux remous que les bulles d'air provoque dans la biomasse contenue dans le container. A l'issue de cette étape, la biomasse solide partiellement ou totalement digérée vient reposer sur le fond du container.

De façon alternative, la vidange aérobie du digestat liquide peut se faire sans utilisation de la pompe de vidange du digesteur. Dans ce cas de figure la vidange aérobie est réalisée de la façon suivante : la vanne, apte à obturer ou permettre le passage de l'air entre l'intérieur et l'extérieur de la cuve du digesteur est fermée, de même la vanne permettant d'obturer ou de faire circuler le biogaz et le digestat liquide est fermée, un circuit de tuyauterie permettant de contourner la pompe d'évacuation du digestat liquide est ouvert, puis de l'air est injecté dans le digesteur, dans la partie inférieure de la cuve du digesteur, au moyen du compresseur d'air, ce qui a pour effet que l'air s'accumule dans la partie supérieure de la cuve du digesteur, chassant par pression le digestat liquide par la partie inférieure de la cuve du digesteur, lequel digestat liquide est transféré vers l'unité de digestion principale par le circuit de tuyauterie permettant de contourner la pompe d'évacuation du digestat liquide.

Le procédé de production du biogaz présente donc l'avantage de ne pas nécessiter une étape de déchargement de la biomasse solide contenue dans le container, de préparation et/ou de séparation des éléments indésirables et/ou de broyage et/ou de dilution préalable de la biomasse solide ni de nécessiter de brassage de la biomasse solide dans le digesteur, ce qui permet de minimiser les investissements en équipements et les dépenses énergétiques associées à de telles étapes ainsi que les risques éventuels d'endommagement des équipements du digesteur ou des équipements mis en œuvre au cours du procédé et de diminuer les coûts de personnel et de maintenance liés aux différentes étapes évitées.

Selon une caractéristique, entre l'étape de digestion de la biomasse solide et l'étape de vidange aérobie du digestat liquide, le procédé comprend au moins les étapes suivantes :
- une étape de vidange anaérobie du digestat liquide du container et de la cuve du digesteur, et
- une étape de re-remplissage de la cuve du digesteur avec un digestat liquide après vidange anaérobie.

Selon une caractéristique, entre l'étape de re-remplissage de la cuve du digesteur avec un digestat liquide et l'étape de vidange aérobie de la biomasse liquide, le procédé comprend en outre au moins une étape de digestion constituée par une percolation du digestat liquide à travers la biomasse solide contenue dans le container et d'une diffusion du digestat liquide dans la biomasse solide pour générer puis récupérer le biogaz à travers la zone d'évacuation du biogaz située dans la partie supérieure du digesteur.

En particulier, le procédé comporte une étape d'ouverture du toit du digesteur avant l'étape d'introduction du container afin de permettre l'introduction par le haut de la cuve du digesteur du container contenant de la biomasse solide.

La zone d'admission est ainsi située sur au moins une partie de la surface supérieure de la cuve sur laquelle le toit se referme.

Selon un mode de réalisation, le procédé comporte une étape de fermeture du toit sur la zone d'admission du digesteur préalablement à l'étape de remplissage de la cuve avec du digestat liquide.

Optionnellement, le système d'accrochage peut également ouvrir et éventuellement fermer le toit du digesteur.

Le digestat liquide est ensuite introduit à l'intérieur de la cuve par le biais d'une ou plusieurs pompes d'alimentation en provenance de l'unité de digestion principale apte à stocker du biogaz et du digestat liquide.

Selon un mode de réalisation, après l'étape d'introduction dans la cuve du container contenant la biomasse solide à traiter puis le remplissage de la cuve par du digestat liquide, la vanne, apte à obturer ou permettre le passage de l'air entre l'intérieur et l'extérieur de la cuve du digesteur, est fermée de manière à ce que la réaction de méthanisation se déroule dans des conditions anaérobies.

L'étape de percolation conduit au niveau de la partie supérieure du digesteur, à la formation d'un flux de biogaz et de digestat liquide.

La percolation est assurée par la mise en œuvre d'une ou plusieurs pompes de circulation située(s) entre l'unité de digestion principale et la cuve du digesteur.

En particulier, la ou les pompe(s) de circulation permet(tent) d'assurer un courant et une pression du digestat liquide sur la biomasse solide contenue dans le container obligeant le digestat le digestat liquide à la traverser la biomasse solide, ce qui favorise un contact intime entre les bactéries contenues notamment dans le digestat liquide et la biomasse solide à dégrader.

La percolation s'accompagne d'une diffusion du digestat liquide dans la biomasse solide, laquelle est favorisée par la pression exercée par le pompage du digestat liquide dans la biomasse solide. Cette diffusion favorise le contact intime entre les bactéries et la biomasse solide à traiter.

De plus, le pompage et la circulation forcée du digestat liquide entre l'unité de digestion principale et le digesteur présentent l'avantage de faire circuler une très grande quantité de digestat liquide à travers un volume plus faible de biomasse solide à traiter, ce qui limite les risques d'acidose dans le container situé dans le digesteur, aux alentours de la biomasse solide à traiter.

En effet, dans les autres procédés de méthanisation, des quantités trop importantes de biomasse à traiter par rapport à la quantité de bactéries présentes dans le digesteur peuvent conduire à une réaction d'acidogénèse excessive, pouvant provoquer une acidification du digestat liquide, ou acidose, laquelle entraine une inhibition partielle, voire totale, de la méthanogénèse et donc de la production de méthane. En synthèse, une telle réaction est provoquée lorsque la production d'acides gras volatils, induite par des bactéries acidogènes, est plus importante que la capacité de transformation de ces acides gras en méthane par les bactéries méthanogènes.

Selon un mode de réalisation, l'étape de vidange anaérobie du digestat liquide est mise en œuvre en vidant au niveau de la partie inférieure de la cuve du digestat liquide ce qui provoque le tassement de la biomasse solide au fond du container et permet d'éliminer les chemins préférentiels de percolation ainsi que les poches de biogaz bloquées dans la biomasse.

L'étape de vidange anaérobie peut être mise en œuvre en aspirant le digestat liquide par le biais de la ou les pompe(s) située(s) entre la cuve du digesteur et l'unité de digestion principale.

L'aspiration du digestat liquide peut de préférence avoir lieu en aval de l'élément perforé inférieur pour éviter que la biomasse solide éventuellement échappée du container soit entrainée avec le digestat liquide vers l'unité de digestion principale.

L'étape de vidange anaérobie permet au biogaz en provenance de l'unité de digestion principale de remplir l'ensemble du volume libéré dans la cuve en remplacement du digestat liquide qui est aspiré par la pompe vers l'unité de digestion principale.

Selon un mode de réalisation l'étape de vidange anaérobie peut être accompagnée d'une injection de biogaz en provenance de l'unité de digestion principale dans la partie inférieure de la cuve du digesteur au moyen d'un compresseur de biogaz.

Selon un mode de réalisation, l'injection de biogaz en provenance de l'unité de digestion principale dans la partie inférieure de la cuve du digesteur au moyen d'un compresseur de biogaz peut se faire en dehors des périodes de vidange anaérobie.

Après l'étape de vidange anaérobie, une étape de re-remplissage de la cuve avec du digestat liquide est à nouveau mise en œuvre afin de poursuivre la réaction de méthanisation.

De la même façon que précédemment, l'étape de digestion conduit à la formation de biogaz, récupéré par le biais de la tuyauterie d'évacuation du mélange biogaz/digestat liquide vers l'unité de digestion principale situé au niveau de la partie supérieure du digesteur.

Selon un mode de réalisation, l'étape de vidange aérobie du digestat liquide de la cuve du digesteur est réalisée à la fin du cycle de méthanisation.

Une telle étape est mise en œuvre en fermant la vanne permettant d'obturer ou de faire circuler le biogaz et le digestat liquide, et en ouvrant la vanne permettant d'obturer ou d'introduire l'air dans le digesteur et en vidant la cuve du digestat liquide depuis le bas de la cuve du digesteur vers l'unité de digestion principale.

Selon un mode de réalisation durant l'étape de vidange aérobie, l'aspiration du digestat liquide du digesteur vers l'unité de digestion principale peur être précédée d'une injection d'air dans la partie inférieure de la cuve du digesteur afin de purger l'intérieur du container des éventuelles bulles de biogaz qui pourraient être y restées piégées dans la biomasse digérée, et plus généralement de purger le digesteur du biogaz qui s'y trouve avant la vidange aérobie. Le mélange biogaz-air ainsi récupéré pourra être envoyé dans la réserve de biogaz si la quantité d'air injectée est très faible, ou bien devra de préférence être envoyé et brûlé dans une torchère afin de ne pas être rejeté à l'atmosphère si la quantité d'air injecté est importante.

L'étape de vidange aérobie est mise en œuvre en aspirant le digestat liquide par le biais de la ou les pompe(s) située(s) entre la cuve du digesteur et l'unité de digestion principale.

L'aspiration du digestat liquide peut avoir lieu de préférence en aval de l'élément perforé pour éviter que de la biomasse solide éventuellement échappée du container soit entrainée avec le digestat liquide vers l'unité de digestion principale.

L'étape de vidange aérobie permet à l'air en provenance de l'extérieur de remplir l'ensemble du volume libéré dans la cuve en remplacement du digestat liquide qui est aspiré par la pompe vers l'unité de digestion principale.

Selon un mode de réalisation, la vidange aérobie du digestat liquide peut se faire de façon alternative sans utilisation de la pompe de vidange du digesteur. Une telle étape est réalisée de la façon suivante : la vanne, apte à obturer ou permettre le passage de l'air entre l'intérieur et l'extérieur de la cuve du digesteur est fermée, de même la vanne permettant d'obturer ou de faire circuler le biogaz et le digestat liquide est fermée, un circuit de tuyauterie permettant de contourner la pompe d'évacuation du digestat liquide est ouvert, puis de l'air est injecté dans le digesteur, dans la partie inférieure de la cuve du digesteur, au moyen d'un compresseur d'air, ce qui a pour effet que l'air s'accumule dans la partie supérieure de la cuve du digesteur, chassant par pression le digestat liquide par le bas de la cuve du digesteur, lequel digestat liquide est transféré vers l'unité de digestion principale par le circuit de tuyauterie permettant de contourner la pompe d'évacuation du digestat liquide.

Selon un mode de réalisation, le procédé comporte une étape d'ouverture du toit sur la zone d'introduction du container contenant la biomasse solide, préalablement à l'étape d'évacuation du container contenant le digestat solide, correspondant à la biomasse solide digérée résiduelle après méthanisation.

L'étape d'évacuation du container contenant la biomasse solide digérée résiduelle peut être effectuée par le biais du système d'accrochage apte à saisir ledit container.

Une telle étape présente l'avantage de pouvoir se dérouler en toute sécurité après la vidange aérobie du digestat liquide, en absence de contact avec le biogaz, lequel biogaz a été évacué pendant l'étape de vidange aérobie.

L'invention concerne encore un système de production de biogaz, comportant au moins un digesteur tel que défini précédemment, qui est ou sont relié(s) par le biais d'un circuit d'alimentation en digestat liquide et d'un circuit d'évacuation de biogaz et de digestat liquide, à au moins une unité de digestion principale apte à contenir du biogaz et du digestat liquide.

En d'autres termes, le système de production de biogaz correspond à une installation de méthanisation mettant en œuvre une unité de digestion principale destinée à alimenter en digestat liquide un ou plusieurs digesteurs de méthanisation, tel(s) que défini(s) précédemment, et à récupérer le biogaz produit par ce ou ces différents digesteurs.

Alternativement, la ou les unités de digestion principale peuvent être constituées chacune par deux ouvrages distincts, l'un destiné à contenir principalement du digestat liquide, l'autre destiné à contenir principalement du biogaz.

De préférence, le circuit d'évacuation de biogaz et de recirculation du digestat liquide vers l'unité de digestion principale comprend un circuit apte à acheminer du biogaz et un circuit apte à acheminer du digestat liquide.

De préférence, le circuit d'évacuation comprend un pot de séparation apte à séparer le biogaz et le digestat liquide.

L'unité de digestion principale ne contient pas de biomasse solide ou dans des quantités très faibles.

L'unité de digestion principale peut avoir une fonction complémentaire de production de biogaz. En effet, durant l'étape de digestion, la recirculation du digestat liquide en provenance du ou des digesteurs vers l'unité de digestion principale, après percolation du digestat liquide dans la biomasse solide, peut provoquer des entrainements avec le digestat liquide de matières fines solides non encore digérées et/ou des entrainements d'acide gras volatiles qui n'ont pas eu le temps d'être transformés en biogaz dans le digesteur. Dans ces conditions, la méthanisation résiduelle de ces matières se produit dans l'unité de digestion principale.

Comme indiqué précédemment, l'unité de digestion principale peut être composée d'un ou plusieurs ouvrages.

L'invention a également pour objet un container apte à être introduit dans la cuve du digesteur, tel que défini précédemment, comprenant un élément de séparation perforé supérieur et un élément de séparation perforé inférieur, aptes à laisser s'introduire du digestat liquide à l'intérieur du container et à retenir la biomasse solide digérée ou non.

Ainsi le container est équipé d'un système permettant, lorsque le container se trouve dans le digesteur, de libérer le passage du digestat liquide à travers la biomasse solide contenue dans le container, tout en étant capable de retenir la biomasse solide dans ledit container.

Selon une caractéristique de l'invention, le container comprend un système d'ouverture et de fermeture, situé entre la surface inférieure du container et l'élément de séparation perforé inférieur, apte à s'ouvrir lorsque le système d'ouverture et de fermeture entre en contact avec le fond de la cuve du digesteur.

De façon alternative, le système d'ouverture et de fermeture peut être situé sous la surface inférieure du container en excroissance vers l'extérieur du container.

En conséquence, le container comprend un système d'ouverture et de fermeture fixé sur la surface inférieure du container, ledit système étant apte à s'ouvrir au contact avec le fond de la cuve du digesteur
Ainsi le système d'ouverture et de fermeture correspond à un système de trappe étanche située au niveau de la surface inférieure du container qui est en position fermée lorsque le container se trouve en dehors du digesteur et qui est automatiquement en position ouverte lorsque le container est en contact avec le fond de la cuve du digesteur.

Selon une caractéristique de l'invention, le container comprend un système d'ouverture et de fermeture, situé entre l'élément de séparation perforé supérieur et la surface supérieure du container, apte à s'ouvrir lorsque le système d'ouverture et de fermeture entre en contact avec le toit du digesteur.

De façon alternative, le système d'ouverture et de fermeture peut être situé sur la surface supérieure du container en excroissance vers l'extérieur du container.

En conséquence, le container comprend un système d'ouverture et de fermeture fixé sur la surface supérieure du container, ledit système étant apte à s'ouvrir au contact avec le toit du digesteur

Ainsi le système d'ouverture et de fermeture correspond à un système de trappe étanche située au niveau de sa surface supérieure qui est en position fermée lorsque le container se trouve en dehors du digesteur et qui est automatiquement en position ouverte lorsque le container est en contact avec le toit surmontant la cuve du digesteur.

Le digesteur selon l'invention est apte à recevoir un container tel que décrit précédemment.

D'autres avantages et caractéristiques de l'invention apparaîtront à l'examen de la description détaillée d'un mode de réalisation de l'invention nullement limitatif, et des dessins annexés, sur lesquels :
- la figure 1 représente schématiquement une vue en coupe du digesteur, selon l'invention, relié à une unité de digestion principale stockant conjointement du biogaz et du digestat liquide,
- les figures 2 à 9 représentent schématiquement des vues en coupe du digesteur au cours des différentes étapes du procédé de production du biogaz,
- la figure 10 représente schématiquement une vue d'un container vide,
- la figure 11 représente schématiquement une vue du système d'ouverture et de fermeture du container,
- la figure 12 représente schématiquement une vue de dessus d'un digesteur selon l'invention,
- la figure 13 illustre schématiquement une vue de dessus d'un système de production de biogaz et de digestat comprenant une unité de digestion principale apte à alimenter en digestat liquide une pluralité de digesteurs, conformes à la présente invention, et à stocker le biogaz en provenance desdits digesteurs.

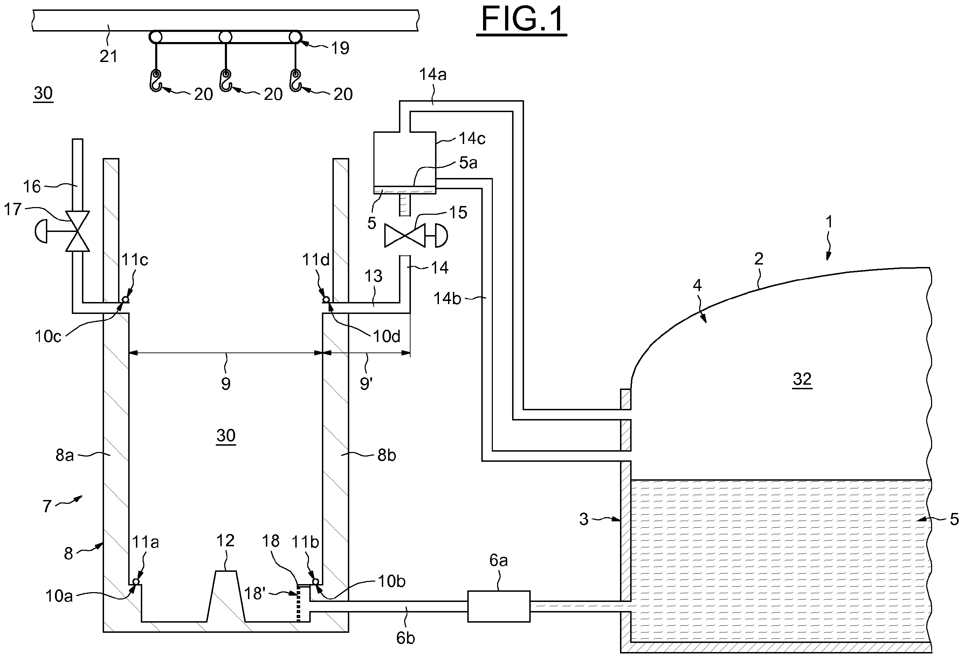

Sur la figure 1 est représentée de manière schématique, une unité de digestion principale 1, composée d'un seul ouvrage, et un digesteur 7 selon l'invention.

L'unité de digestion principale 1 comprend une cuve 3, ayant une forme essentiellement cylindrique, remplie de digestat liquide 5, qui est surmontée d'une membrane étanche 2 constituant un espace de stockage de gaz 4, lequel est rempli de biogaz 32. La membrane 2 occupe donc la partie en hauteur de l'unité de digestion principale 1 formant ainsi un dôme contenant un ciel gazeux. La cuve 3 correspond à une réserve de digestat liquide 5.

En variante, la cuve 3 peut présenter une forme cubique ou parallélépipédique.

La cuve 3 peut être fabriquée à partir d'acier, de béton ou de tout autre matériau tandis que la membrane étanche 2 peut être réalisée en polymère, notamment en polyéthylène, polypropylène ou chlorure de polyvinyle.

En variante, la membrane étanche 2 peut être surmontée par au moins une membrane supplémentaire de manière à ce que de l'air soit pris en sandwich entre la membrane supplémentaire et la membrane étanche 2.

L'unité de digestion principale 1 est reliée à un digesteur 7, selon l'invention, par l'intermédiaire d'une tuyauterie 6b sur laquelle est montée une pompe 6a. Ainsi les parties inférieures de l'unité de digestion principale 1 et du digesteur 7 sont reliées entre elles grâce à la tuyauterie 6b.

Le digesteur 7 comprend une cuve 8, de forme essentiellement parallélépipédique, de préférence de forme parallélépipédique rectangle, dotée de parois 8a et 8b, pouvant être des murs en béton, et d'un orifice d'entrée 9, ayant une surface inférieure à la surface totale de la partie supérieure de la cuve 8. Les parois 8a et 8b présentent au niveau de la partie inférieure de la cuve 8 des renfoncements formant des rebords à angle droit 10a et 10b respectivement, situés à la même hauteur, et sur lesquels reposent préférentiellement des joints d'étanchéité 11a et 11b. Les parois 8a et 8b sont donc plus épaisses en dessous des rebords à angle droit 10a et 10b.

Le fond de la cuve 8 comprend, de préférence en son centre, un élément en saillie 12, de forme essentiellement conique, dont la hauteur est supérieure à la hauteur des rebords à angle droit 10a et 10b.

Les parois 8a et 8b présentent également, au niveau de la partie supérieure de la cuve 8, un rétrécissement formant respectivement des rebords à angle droit 10c et 10d et sur lesquels reposent préférentiellement des joints d'étanchéité 11c et 11d. Ainsi l'épaisseur des parois 8a et 8b n'est pas identique au dessus et en dessous des rebords à angle droit 10c et 10d. En particulier, l'épaisseur des parois 8a et 8b est plus fine au dessus des rebords 10c et 10d.

En variante, la cuve 8 peut également présenter une forme essentiellement cylindrique.

De manière générale, la cuve 8 est configurée de manière à recevoir un container 22.

La pompe 6a permet non seulement d'alimenter le digesteur 7 en digestat liquide 5 provenant de l'unité de digestion principale 1 mais également de vidanger le digestat liquide 5 se trouvant à l'intérieur de la cuve 8 et du container 22, une fois introduit dans la cuve 8, vers l'unité de digestion principale 1. Dans le mode de réalisation illustré dans la figure 1, la tuyauterie 6b relie la cuve 3, remplie en digestat liquide 5, de l'unité de digestion principale 1, à la cuve 8 du digesteur 7, qui est vide.

La cuve 8 comporte, au niveau de sa partie supérieure, une tuyauterie 13 d'évacuation par débordement du mélange digestat liquide/biogaz, qui débouche au niveau du rebord à angle droit 10d de la cuve 8. Cette tuyauterie 13 est encastrée dans la partie supérieure de la paroi 8b et est en saillie vers l'intérieur de la cuve 8 par rapport à la paroi 8b formant ainsi un rebord à angle droit avec la partie supérieure de la paroi 8b.

Ainsi la partie supérieure de la paroi 8b présente, au-dessus de la tuyauterie 13, une épaisseur plus faible, qu'en-dessous de la tuyauterie 13.

L'entrée de la tuyauterie de débordement 13 est connectée à une conduite 14 sur laquelle est montée une vanne 15 située en amont d'un pot de séparation 14c, lequel est relié à deux circuits distincts 14a et 14b qui sont reliés à leur extrémité à l'unité de digestion principale 1. La conduite 14 permet de récupérer un fluide issu de la tuyauterie de débordement 13 et la vanne 15 permet d'obturer ou de permettre la circulation du fluide dans le circuit d'acheminement 14. La vanne 15 se trouve en position fermée sur la figure 1.

Le circuit 14a, destiné à acheminer principalement le biogaz, se trouve au-dessus du circuit 14b, destiné à acheminer principalement le digestat liquide, ce qui facilite le transfert des différents fluides.

Le pot 14c, monté sur le circuit d'acheminement 14, permet de séparer plus efficacement le biogaz du digestat liquide lorsqu'ils sont extraits de la cuve 8 à travers la tuyauterie 13.

Dans la figure 1, le pot 14c contient du digestat liquide 5 issu d'un précédent cycle de méthanisation.

Le circuit 14b peut comprendre une pompe de circulation (non représenté sur la figure 1) afin de faciliter l'acheminement du digestat liquide.

La tuyauterie de débordement 13 se trouve donc localisé dans une zone 9' d'évacuation du biogaz, située au niveau de la partie supérieure de la cuve 8 du digesteur 7.

Sur la figure 1, n'a été représenté, en coupe, qu'une tuyauterie de débordement 13 mais, de préférence, une pluralité de tuyauteries de débordement 13a, 13b, 13c etc. peuvent être raccordées à la conduite 14.

La cuve 8 comporte également, au niveau de sa partie supérieure, une tuyauterie 16 qui est en saillie vers l'extérieur et qui débouche au niveau du rebord à angle droit 10c de la cuve 8. Ainsi la tuyauterie 16 forme un rebord à angle droit avec la partie supérieure de la paroi 8a.

Ainsi la partie supérieure de la paroi 8a présente, au-dessus de la tuyauterie 16, une épaisseur plus faible, qu'en-dessous de la tuyauterie 16.

La tuyauterie 16 est ouverte vers l'extérieur ce qui permet d'acheminer l'air à l'intérieur de la cuve 8 du digesteur 7 ou de l'évacuer vers l'extérieur de la cuve 8. La vanne 17, montée sur la tuyauterie 16, permet de rendre possible ou d'empêcher l'introduction ou l'évacuation de l'air à l'intérieur de la cuve 8.

Sur la figure 1 est représenté l'air 30 qui est à la fois localisé à l'extérieur et à l'intérieur de la cuve 8 du digesteur 7.

La cuve 8 comporte également, au niveau de sa partie inférieure, un élément de séparation perforé inférieur 18, de préférence une plaque perforée recouvrant l'extrémité de la tuyauterie 6b d'injection dans le digesteur du digestat liquide en provenance de l'unité de digestion principale 1. En alternative, l'élément de séparation perforé inférieur 18 peut être un tuyau perforé en saillie vers l'intérieur de la cuve 8 du digesteur 7, dans le prolongement de la tuyauterie 6b d'injection dans le digesteur 7 du digestat liquide 5 en provenance de l'unité de digestion principale 1. L'élément de séparation 18 comprend une pluralité d'interstices 18' afin de laisser circuler le digestat liquide 5 lors des étapes de vidanges anaérobies et aérobies du digesteur 7 tout en retenant la biomasse solide 23 ou le digestat solide 23' qui se seraient éventuellement échappés hors du container 22 situé dans la cuve 8 du digesteur 7.

Sur la figure 1, un système d'accrochage 19 comportant trois crochets 20 est positionné au-dessus de l'orifice d'entrée 9 de la cuve 8 et chacun de ces crochets est suspendu au système d'accrochage 19, situé sur un pont roulant 21, par l'intermédiaire d'un câble ou d'un filin pouvant être en acier. Le système d'accrochage 19, tel que représenté, est piloté à partir du pont roulant 21.

Sur la figure 1, la cuve 8 du digesteur 7 a été représentée ouverte sans la présence d'un toit amovible 24.

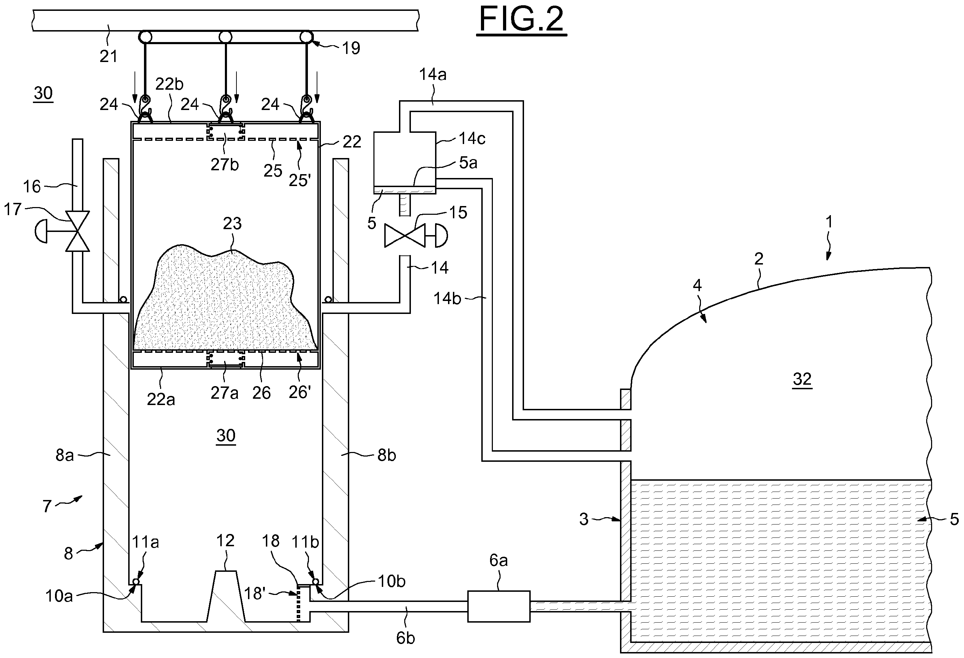

Les figures 2 à 9 représentent les étapes successives d'un procédé de production de biogaz conforme à l'invention.

Sur la figure 2 est représentée schématiquement l'étape de d'introduction dans la cuve 8 du digesteur 7 d'un container 22 contenant de la biomasse solide 23, en particulier hétérogène et/ou fibreuse, à l'aide du système d'accrochage 19.

Au cours de cette étape, le toit amovible 24 du digesteur 7 a été préalablement enlevé par le système d'accrochage et repose sur une aire de repos prévue à cet effet (étape non représentée sur la figure 2).

Le système d'accrochage 19 permet de saisir le container 22 contenant la biomasse solide 23, qui était situé à l'extérieur du digesteur 7, par le biais d'anses 24, qui sont situées au niveau de la surface supérieure 22b du container 22, afin de le déposer à l'intérieur de la cuve 8 du digesteur 7. En d'autres termes, le système d'accrochage 19 permet d'introduire, par le haut de la cuve 8 du digesteur 7, le container 22 contenant la biomasse solide 23, à travers l'orifice d'entrée 9 qui est situé au niveau de la partie supérieure de la cuve 8 du digesteur 7. Ainsi l'orifice d'entrée 9 constitue une zone d'introduction du container 22 contenant la biomasse solide 23. De plus, le container 22 reste de préférence étanche lors de cette étape d'introduction dans le digesteur 7.

Le système d'accrochage 19 permet de saisir le container 22 contenant la biomasse solide 23, laquelle biomasse solide contient éventuellement des éléments indésirables, tels que des sacs ou des morceaux de plastique, des restes d'emballage, des boites de conserve et autres objets métalliques, des objets en verre ou des morceaux de verre, des morceaux de tissu, des ficelles, des cailloux, du gravier, du sable, des barbelés, des pièces métalliques provenant d'outils agricoles, sans avoir besoin d'effectuer d'opération de déchargement/chargement préalable, ni de tri, ni de broyage, ni de maintient en suspension forcé de la biomasse solide dans un liquide.

Conformément à la figure 2, le container 22 présente, d'une part, au niveau de sa partie supérieure, un élément de séparation supérieure 25, de préférence une grille, percé de plusieurs orifices 25' ayant des tailles identiques ou différentes, et, d'autre part, au niveau de sa partie inférieure, un élément de séparation inférieure 26, de préférence une grille, percé de plusieurs orifices 26' ayant des tailles identiques ou différentes. La biomasse solide 23 repose ainsi sur l'élément de séparation inférieure 26. Les orifices 25' et 26' sont configurés de façon à ce que la biomasse solide 23 ne puisse pas traverser les éléments de séparation 25 et 26 au cours des différentes étapes du procédé.

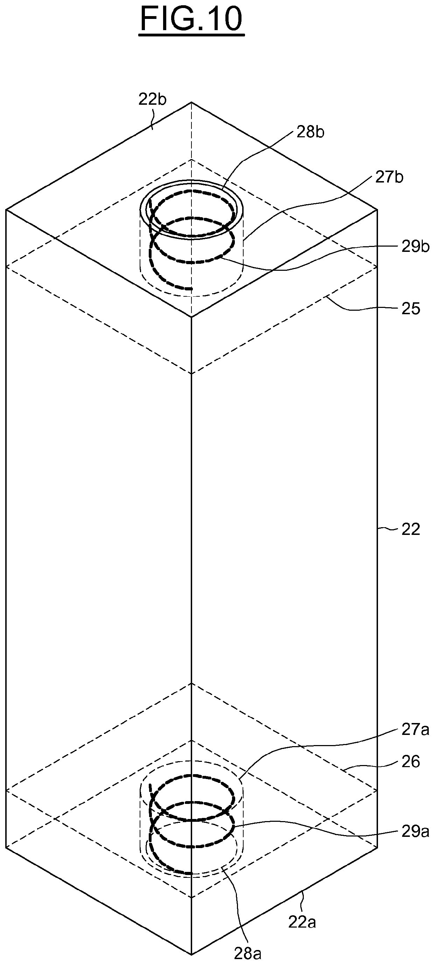

Le container 22 est illustré plus en détails dans la figure 10. En effet, le container 22 comprend, d'une part, un système d'ouverture et de fermeture 27a qui est fixé, par exemple au moyen d'une soudure entre la surface inférieure 22a du container (correspondant au fond du container) et l'élément de séparation inférieur 26 muni d'orifices 26' et, d'autre part, un système d'ouverture et de fermeture 27b entre la surface supérieure 22b du container (correspondant au toit du container) et l'élément de séparation supérieur 25 muni des orifices 25'. Ainsi le système d'ouverture et de fermeture 27a relie la surface inférieure 22a et l'élément de séparation inférieur 26 et le système d'ouverture et de fermeture 27b relie la surface supérieure 22b à l'élément de séparation supérieur 25.

De préférence, le système d'ouverture et de fermeture 27a est positionné au centre de la surface inférieure 22a et le système d'ouverture et de fermeture 27b est positionné au centre de la surface supérieure 22b.

De préférence, les systèmes d'ouverture et de fermeture 27a et 27b sont identiques et alignés entre eux.

De préférence, le système d'ouverture et de fermeture 27a correspond à un cylindre perforé sur l'ensemble de sa paroi par une pluralité d'orifices 27a' ayant pour fonction de faciliter le passage du digestat liquide 5. En particulier, le système d'ouverture et de fermeture 27a est un cylindre perforé ayant des bases soudées à la surface inférieure 22a et l'élément de séparation inférieur 26. Il en va de même pour le système d'ouverture et de fermeture 27b.

Le système d'ouverture et de fermeture 27a comprend également un élément d'obstruction 28a, préférentiellement en forme de disque, relié à un ressort 29a. L'élément d'obstruction 28a permet d'obstruer un trou 30a situé au niveau de la surface inférieure 22a du container 22.

De la même façon, le système d'ouverture et de fermeture 27b comprend également un élément d'obstruction 28b, préférentiellement en forme de disque, relié à un ressort 29b. L'élément d'obstruction 28b permet d'obstruer un trou 30b situé au niveau de la surface supérieure 22b du container 22.

Ainsi les éléments d'obstruction 28a et 28b permettent d'assurer l'étanchéité du container 22 en obstruant respectivement les trous 30a et 30b lorsqu'aucun élément externe ne vient exercer une pression sur lesdits éléments d'obstruction 28a et 28b. En d'autres termes, le container est étanché à l'extérieur du digesteur 7.

A la suite de l'étape d'introduction décrite à la figure 2, le container 22 se trouve à l'intérieur de la cuve 8 du digesteur, en particulier positionné sur les rebords à angle droit 10a et 10b de la cuve 8 du digesteur 7, et plus particulièrement sur les joints d'étanchéité 11a et 11b.

De plus, le container 22 est positionné à l'intérieur de la cuve 8 de manière à ce que l'élément en 12 se trouve en regard du système d'ouverture et de fermeture 27a. Autrement dit, le container 22 est positionné à l'intérieur de la cuve 8 de manière à ce que l'élément en saillie 12 soit aligné avec le système d'ouverture et de fermeture 27a.

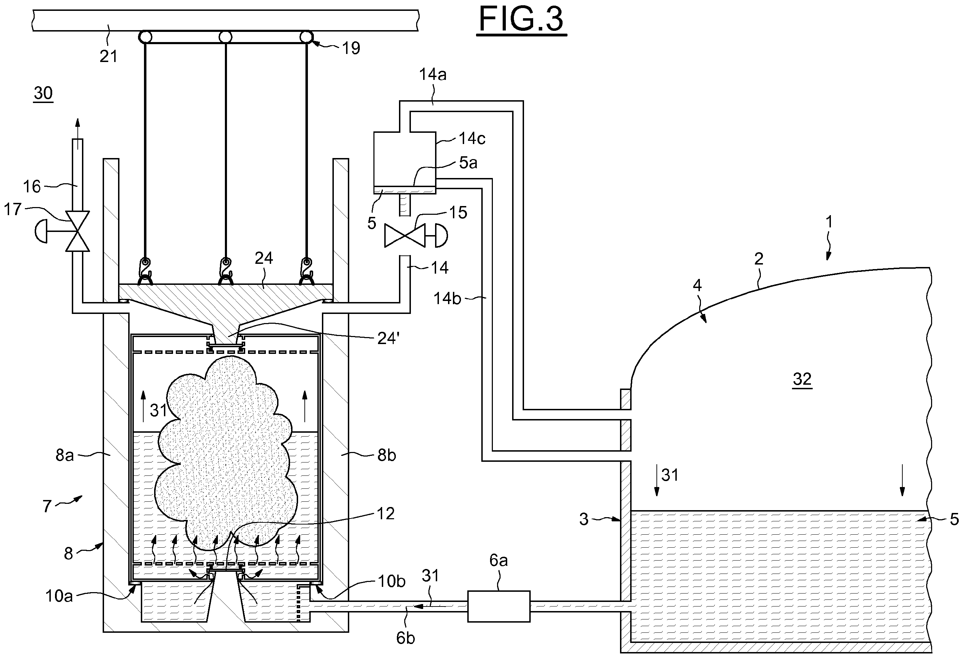

Comme le montre la figure 3, lorsque l'étape d'introduction dans la cuve 8 du container 22 contenant la biomasse solide 23 est terminée, un toit amovible 24 est fermé de manière à recouvrir complètement de façon étanche l'orifice d'entrée 9 du digesteur 7.

En particulier, les bords du toit amovible 24 reposent sur les rebords à angle droit formés entre les tuyauteries 13 et 16 et les parois 8a et 8b respectivement.

De préférence, le toit amovible 24 comporte un élément en saillie 24' qui est positionné en regard du système d'ouverture et de fermeture 27b. Autrement dit, le toit amovible 24 se referme sur l'orifice d'entrée 9 du digesteur 7 de manière à ce que l'élément en saillie 24' soit aligné avec le système d'ouverture et de fermeture 27b.

Ainsi le toit amovible 24 est configuré pour pouvoir se refermer sur l'orifice d'entré 9 de la cuve 8 du digesteur 7.

Conformément à la figure 3, après introduction du container 22 contenant la biomasse solide 23, et après fermeture du toit amovible 24, la cuve 8 est remplie avec du digestat liquide 5, qui est acheminé par le biais de la pompe d'alimentation 6a, en provenance de la cuve 3 de l'unité de digestion principale 1. En particulier, sur la figure 3, le digestat liquide 5 est introduit au niveau de la partie inférieure de la cuve 8 et circule à travers les interstices 18' de l'élément de séparation perforé 18 de la cuve 8.

La cuve 8 du digesteur 7 peut également être remplie avec du digestat liquide 5 à travers une entrée située dans une zone adjacente ou située au dessus de l'élément perforé inférieur 18.

Ainsi la cuve 8 peut être remplie avec du digestat liquide 5 qui ne passe pas à travers l'élément perforé inférieur 18.

Au cours de cette étape, l'élément en saillie 12 exerce une pression sur l'élément d'obstruction 28a qui est situé entre la surface inférieure 22a et l'élément de séparation inférieur perforé 26.

De cette façon, l'élément en saillie 12 pénètre dans le trou 30a de la surface inférieure 22a en repoussant l'élément d'obstruction 28a vers le haut, c'est-à-dire verticalement vers l'élément de séparation inférieur perforé 26. Ainsi l'élément en saillie 12 exerce une pression sur l'élément d'obstruction 28a qui va compresser le ressort 29a. En d'autres termes, l'élément en saillie 12 va légèrement pénétrer dans le container 22 par le biais du système d'ouverture et de fermeture 27a.

Le digestat liquide 5, en provenance de la cuve 3 de l'unité de digestion principale 1 et se trouvant dans le container 22, circule alors au travers des orifices 27a' du système d'ouverture 27a pour ensuite circuler à travers les orifices 26' de l'élément de séparation inférieur perforé 26. De cette façon, le digestat liquide 5 remplit progressivement le container 22.

Par ailleurs, les joints 11a et 11b permettent d'empêcher ou de minimiser la circulation du digestat liquide 5 entre les parois 8a et 8b et les parois du container 22.

Par ailleurs, l'élément en saillie 24' du toit amovible 24 exerce également une pression sur l'élément d'obstruction 28b qui est situé entre la surface supérieure 22b et l'élément de séparation supérieur perforé 25.

De cette façon, l'élément en saillie 24' pénètre dans le trou 30b de la surface supérieure 22b en repoussant l'élément d'obstruction 28b vers le bas, c'est-à-dire verticalement vers l'élément de séparation inférieur 25. En d'autres termes, l'élément en saillie 24' du toit amovible va pénétrer légèrement dans le container 22 par le biais du système d'ouverture et de fermeture 27b.

Au cours de cette étape d'introduction dans la cuve 8 du digestat liquide 5, la vanne 17 est laissée en position ouverte de manière à ce que l'air, présent dans la cuve 8, soit évacué du digesteur 7 par l'intermédiaire de la tuyauterie 16. Sur la figure 3 est représenté schématiquement, l'air 30 qui s'échappe de la tuyauterie 16 pendant le remplissage en digestat liquide 5 de la cuve 8. La vanne 15 est fermée de manière à ce que l'air 30 ne circule pas vers l'unité de digestion principale 1 et que le biogaz 32 ne puisse pas pénétrer dans le digesteur 7 et se mélanger avec l'air 30 présent dans ledit digesteur.

La biomasse solide 23 se retrouve alors immergée dans le digestat liquide 5 à l'intérieur du container 22 contenant la biomasse solide 23.

Le niveau du digestat liquide 5 dans la cuve 3 du digesteur principal 1 diminue tandis qu'il augmente à l'intérieur du container 22 chassant l'air, se trouvant à l'intérieur du container 22, qui s'échappe à travers la tuyauterie 16. L'étape de remplissage sera considérée comme étant terminée lorsque le niveau du digestat liquide 5 aura atteint et légèrement dépassé vers le haut le niveau de la vanne 17, et que la totalité de l'air contenu dans la cuve 8 aura été évacuée.

Sur la figure 3 est représenté, d'une part, le sens de circulation 31 du digestat liquide 5 à l'intérieur du container 22 contenant la biomasse solide 23 qui reflète l'augmentation de niveau dans le container 22 et, d'autre part, le sens de circulation 31 qui illustre la baisse de niveau du digestat liquide 5 à l'intérieur de l'unité de digestion principale 1.

De plus, le sens de circulation 31 du digestat liquide 5 est représenté à l'intérieur de la tuyauterie 6b.

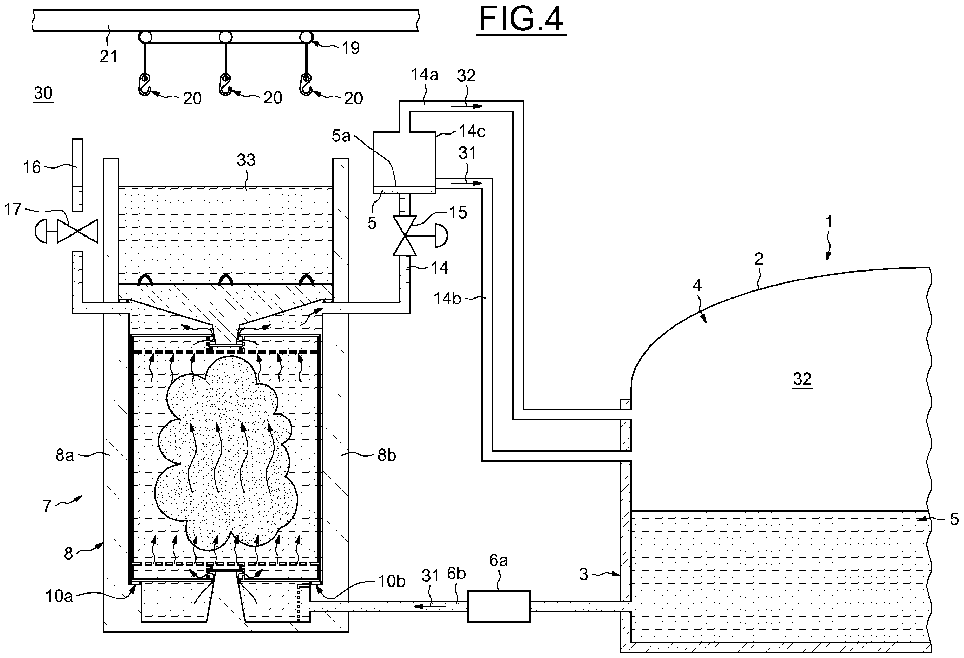

Conformément à la figure 4, une fois la cuve 8 remplie par le digestat liquide 5, la vanne 17 est fermée de manière à placer le digesteur 7 dans des conditions anaérobies, c'est-à-dire à empêcher toute introduction d'air dans la cuve 8, tandis que la vanne 15 est ouverte afin de recueillir le mélange biogaz et digestat liquide.

La figure 4 représente donc l'étape de digestion de la biomasse 23 dans des conditions anaérobies. Au cours de cette étape, du biogaz se forme dans le container 22 contenant la biomasse solide 23, lequel biogaz s'échappe du container 22 à travers l'élément de séparation supérieur 25 du container 22 et passe par flottaison dans la partie supérieure de la cuve 8 pour être conduit ensuite vers l'unité de digestion principale 1.

Une percolation du digestat liquide 5 à travers la biomasse 23 située dans le container 22 est mise en œuvre par le pompage du digestat liquide 5 en provenance de l'unité de digestion principale 1. La percolation se déroule à travers la biomasse solide 23 de manière à ce que la plus grande partie possible de la biomasse solide 23 soit mise en contact intime avec les bactéries contenues dans le digestat liquide 5 et puisse ainsi réagir au cours de la digestion. Dans la figure 4 est représenté schématiquement le flux de digestat liquide 5 circulant, par percolation verticale ascendante, à travers l'ensemble de la biomasse solide 23 contenue dans le container 22.

Conformément à la figure 4, de préférence, l'alimentation en digestat liquide 5 continue au cours de toute l'étape de digestion afin que le digestat liquide 5 continue de circuler à travers la biomasse solide 23, ce qui diminue le risque de survenue de réactions chimiques ou biologiques ponctuelles indésirables en certains endroits du tas de biomasse solide 23, tel que l'acidose par exemple, lesquelles pourraient altérer la qualité de la flore bactérienne.

Cependant, l'alimentation en digestat liquide 5 peut être, dans certains cas, temporairement ou définitivement interrompue, en particulier vers la fin du cycle de méthanisation, moment où les réactions chimiques et biologiques sont moins virulentes.

Au cours de cette étape, un processus de diffusion du digestat liquide 5 dans la biomasse solide 23 se produit. Un tel processus de diffusion est favorisé, d'une part, par l'immersion de la biomasse solide 23 dans le digestat liquide 5 et, d'autre part, par la pression du digestat liquide 5 sur la biomasse solide 23 qui résulte de l'action de la pompe 6a.

Dans le mode de réalisation de la figure 4, la biomasse hétérogène solide 23 peut se désagréger au cours de la percolation du digestat liquide 5 à travers la biomasse solide 23 et de la diffusion du digestat liquide 5 dans la biomasse solide 23.

Le digestat liquide circule au travers des orifices 25' de l'élément de séparation inférieur 25 pour ensuite circuler à travers les orifices 27a' du système d'ouverture 27a. De cette façon, le digestat liquide 5 est extrait pour s'acheminer vers la tuyauterie de débordement 13 tandis que la biomasse solide 23 reste retenue et immergée dans le container 22. Le mélange de biogaz 32 et de digestat liquide 5, circulant à la tuyauterie de débordement 13, est représenté schématiquement sur la figure 4.

Des joints 11c et 11d peuvent être positionnés sur les rebords à angle droit 10c et 10d afin de garantir l'étanchéité au moment de l'extraction verticale du digestat liquide 5.

Le biogaz et le digestat liquide sont acheminés ensemble dans le circuit 14 jusqu'au pot de séparation 14c. Dans la figure 4, après séparation du biogaz 32 et du digestat liquide 5, la tuyauterie 14a achemine principalement le biogaz 32 tandis que la tuyauterie 14b achemine principalement le digestat liquide 5.

Sur la figure 4 est représenté le niveau 5a du digestat liquide 5 dans le pot de séparation 14c. Le digestat liquide 5 se retrouve acheminé dans un circuit d'acheminement 14b correspondant à une conduite de débordement du digestat liquide. Le digestat liquide 5 présent dans le circuit 14b est acheminé jusqu'à la cuve 3, remplie de digestat liquide 5, de l'unité de digestion principale 1. Dans la figure 4, le digestat liquide 5 injecté dans la cuve 8 du digesteur 7, par le biais de la pompe 6, est recirculé par débordement, au moyen du circuit d'acheminement 14b, dans la cuve 3 du digesteur principal 1. Le sens de circulation du digestat liquide 5 est représenté par les flèches 31.

Optionnellement, la présence d'une pompe de circulation, non représentée sur la figure 4, montée sur le circuit 14b permet d'aspirer le digestat liquide 5 à travers la tuyauterie de débordement 13.

Le biogaz 32 est quant à lui acheminé dans le circuit 14a jusqu'à l'unité de digestion principale 1, en particulier à un niveau proche de la membrane étanche 2, lequel niveau est de préférence supérieur au niveau maximal de stockage du digestat liquide 5 dans la cuve 3 de l'unité de digestion principale 1.

Ainsi la tuyauterie de débordement du mélange digestat liquide/biogaz est localisée dans une zone d'évacuation 9' du biogaz 32 qui est situé au niveau de la partie supérieure de la cuve 8 de digestion.

Autrement dit, la zone 9' constitue l'espace d'évacuation du digestat liquide 5 et du biogaz 32.

De plus, la zone 9' constitue l'espace d'évacuation ou d'introduction de l'air dans la cuve 8.

L'étape de digestion, permet de produire du biogaz sans avoir à mettre en œuvre une étape de brassage de la biomasse solide 23 ce qui permet de diminuer les investissements en matériels de brassage et d'agitation et de réduire les dépenses énergétiques et de maintenance généralement afférentes à cette étape.

Conformément à la figure 4, un bouchon 33 peut être positionné sur le toit amovible 24 de la cuve 8 de manière à exercer une pression afin de mieux assurer son étanchéité et sa bonne fermeture au cours de l'étape de digestion.

Le bouchon 33 peut être un bouchon liquide constitué d'eau claire ou de digestat liquide pouvant provenir de la réserve en digestat liquide 5 de l'unité de digestion principale 1 et/ou de fuites dans le toit 24 en provenance de la cuve 8. Le bouchon 33 pourra être recouvert par le biais d'un couvercle, d'un toit fermé à double paroi ou d'un couvercle en plastique, notamment une bâche en plastique. De préférence, le niveau supérieur du bouchon liquide 33 sera le même que le niveau de débordement du digestat liquide 5 dans le pot de séparation 14c, vers la tuyauterie de circulation 14b.

En variante, la séparation du biogaz 32 et du digestat liquide 5 peut s'effectuer sans la présence d'un pot de séparation 14c, voire sans la présence de plusieurs tuyauteries d'acheminement 14a ou 14b. Dans ce cas, la séparation du biogaz 32 et du digestat liquide 5 aurait lieu dans l'unité de digestion principale 1.

Durant l'étape de digestion, la biomasse solide 23 située dans le container est immergée dans le digestat liquide 5 et peut en partie flotter en haut du container, se trouver en partie en suspension au milieu du container ou en partie décanter et se trouver en bas du container.

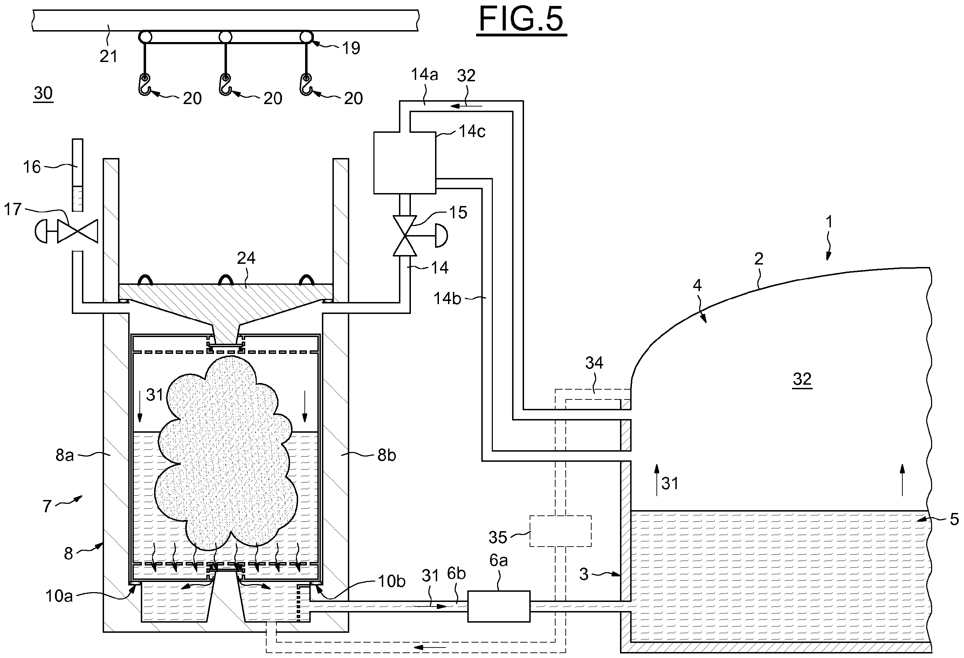

Sur la figure 5 est représentée schématiquement l'étape de vidange anaérobie du digestat liquide 5 de la cuve 8 du digesteur 7, vers la cuve 3 de l'unité de digestion principale 1, en vidangeant le digestat liquide 5 par l'intermédiaire de la pompe 6a et en conservant la vanne 17 en position fermée tandis que la vanne 15 est en position ouverte.

L'étape de vidange anaérobie permet, par le tassement, de la biomasse solide 23 partiellement digérée suite à l'étape de digestion et de diffusion, au fond 22a du container 22, de supprimer les éventuels chemins préférentiels de percolation ainsi que les éventuelles poches de biogaz, bloquées dans la biomasse 23. Ainsi le biogaz 32, se trouvant dans le circuit 14a, circule depuis l'unité de digestion principale 1 en direction de l'intérieur de la cuve 8 du digesteur 7 en passant à travers la tuyauterie de débordement 13 du mélange digestat liquide/biogaz. Le biogaz vient ainsi remplir l'espace laissé vacant par la vidange du digestat liquide 5. Au cours de cette étape de vidange, la biomasse solide 23 redescend vers le fond 22a du container 22 en direction de l'élément de séparation perforé inférieur 25 du container, et vient finalement se déposer sur l'élément de séparation perforé inférieur 25 du container.

Sur la figure 5 est représenté le sens de circulation du biogaz 32 dans le circuit 14 en direction de la cuve 8 ainsi que le sens de circulation 31 du digestat liquide 5 en direction de la cuve 3 de l'unité de digestion principale 1.

L'élément de séparation perforé inférieur 18 présente l'avantage de laisser passer le digestat liquide 5 au moment de la vidange anaérobie en retenant dans la cuve 8 la biomasse solide 23 partiellement digéré qui aurait pu s'échapper du container 22. L'élément de séparation perforé inférieur 18 permet donc de séparer le digestat liquide 5, en le laissant circuler, et la biomasse solide 23 éventuellement échappée du container ce qui permet de ne pas risquer d'obstruer la pompe d'alimentation 6a et de ne pas introduire du digestat solide 23 dans l'unité de digestion principale 1.

Durant l'étape de vidange anaérobie, le bouchon liquide 33 peut être vidangé partiellement ou totalement, notamment pour éviter que s'exerce une pression trop forte sur le toit 24. La partie liquide évacuée peut être vidangée à l'intérieur de la cuve 8 du digesteur 7, ou bien être stockée dans un lieu de stockage pour un usage ultérieur, ou encore être transférée vers une autre cuve de digestion, ou enfin être évacuée vers toute autre destination.

Sur la figure 5 est représenté en pointillés un circuit 34 de compression et d'injection de biogaz reliant l'unité de digestion de principale 1 au niveau de l'espace de stockage 4 de biogaz 32 et la partie inférieure de la cuve 8 du digesteur 7.

Le circuit 34 comprend un compresseur de biogaz 35 permettant d'injecter du biogaz à l'intérieur de la cuve 8 du digesteur lors de l'étape de vidange anaérobie.

Ainsi, l'injection de biogaz pendant l'étape de vidange anaérobie a pour fonction d'agiter la biomasse solide 23 partiellement digérée ce qui permet de favoriser le passage du digestat liquide 5 à travers la biomasse 23 et son évacuation grâce aux remous que les bulles de biogaz provoquent dans la biomasse 23 contenue dans le container 22.

De même, l'injection de biogaz peut aussi être réalisée en dehors de l'étape de vidange anaérobie, pendant l'étape de digestion, ayant alors pour fonction d'agiter la biomasse solide 23 partiellement digérée ce qui permet de supprimer les éventuels chemins préférentiels de percolation ainsi que les éventuelles poches de biogaz bloquées dans la biomasse.

La présence du circuit 34 de compression et d'injection de biogaz lors de l'étape de vidange anaérobie est optionnelle.

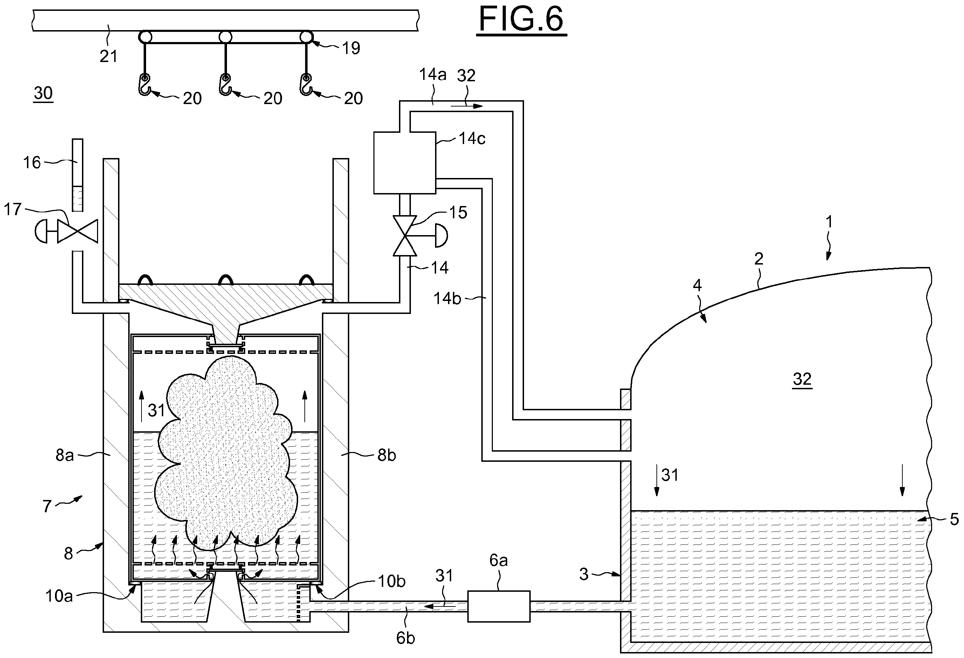

Après la vidange anaérobie de la cuve 8, une nouvelle étape de remplissage de la cuve 8 avec du digestat liquide 5 est mise en œuvre comme représenté sur la figure 6. L'étape de remplissage est identique à la précédente étape de remplissage à l'exception du fait que la vanne 17 est en position fermée et que la vanne 16 est en position ouverte.

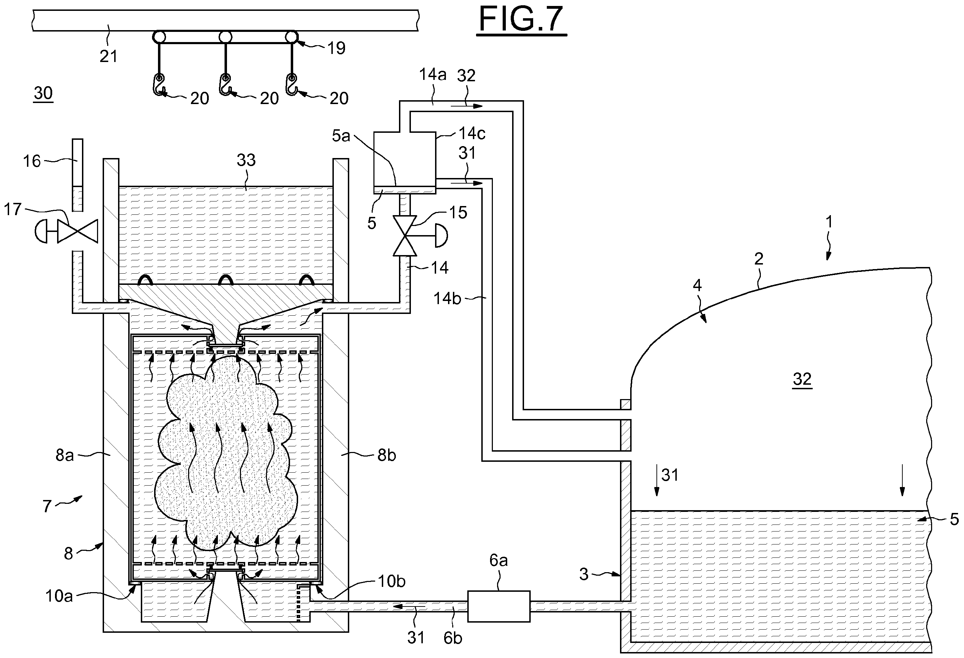

Conformément à la figure 7, une fois l'étape de remplissage terminée, l'étape de digestion de la biomasse solide 23 est une nouvelle fois mise en œuvre afin de continuer la production de biogaz 32.

De la même façon que précédemment, le biogaz 32, issu de l'étape de digestion, est principalement acheminé par le biais du circuit d'acheminement 14a jusqu'à l'unité de digestion principale 1.

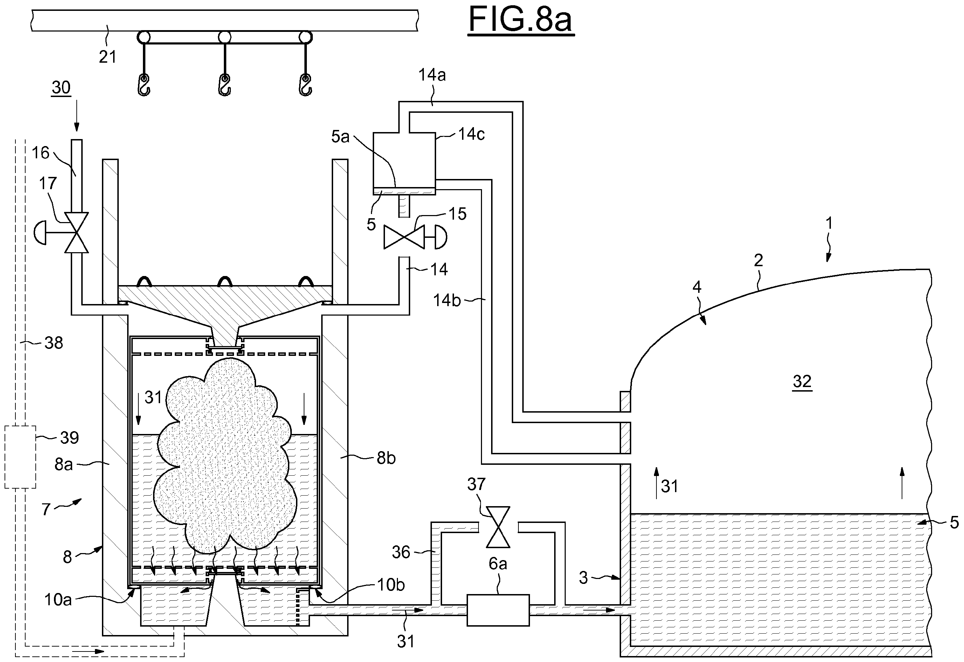

Une fois l'étape de digestion terminée, c'est-à-dire en fin de cycle au bout d'environ 15 à 80 jours, une étape de vidange aérobie de la cuve 8 du digesteur 7 est mise en œuvre comme illustré sur la figure 8a.

L'étape de vidange aérobie est mise en œuvre en fermant la vanne 15 afin d'empêcher toute introduction de biogaz 32 à l'intérieur de la cuve 8 de digestion et du container 22, et en ouvrant la vanne 17 de manière à introduire l'air 30 à l'intérieur de la cuve 8 de digestion en remplacement du digestat liquide 5 évacué et en pompant le digestat liquide 5 par le biais de la pompe 6a à travers l'élément de séparation perforé inférieur 18 afin d'évacuer le digestat liquide 5 vers la cuve 3 de l'unité de digestion principale 1.

Cette étape permet de vidanger le digestat liquide de la cuve 8 de digestion en évitant la présence de biogaz dans la cuve 8 et dans le container 22 situé dans la cuve 8, ce qui permet de minimiser les fuites/rejets de biogaz dans l'atmosphère au moment de la mise en œuvre ultérieure de l'étape d'évacuation du container 22 contenant la biomasse solide 23' résiduelle après digestion, c'est-à-dire le digestat solide.

Sur cette figure, le niveau du digestat liquide 5 dans la cuve 8 diminue et l'air 30 s'introduit progressivement à l'intérieur de la cuve 8 du digesteur 7 en comblant l'espace laissé vacant par la vidange du digestat liquide 5.

Durant cette étape de vidange aérobie, de l'air pourra également être injecté à travers la partie inférieure du digesteur 7 au moyen d'un compresseur d'air pour favoriser le passage du digestat liquide 5 à travers la biomasse 23 et son évacuation grâce aux remous que les bulles d'air provoquent dans la biomasse 23 contenue dans le container 22.

L'air est injecté au moyen d'un compresseur 39 monté sur un circuit 38.

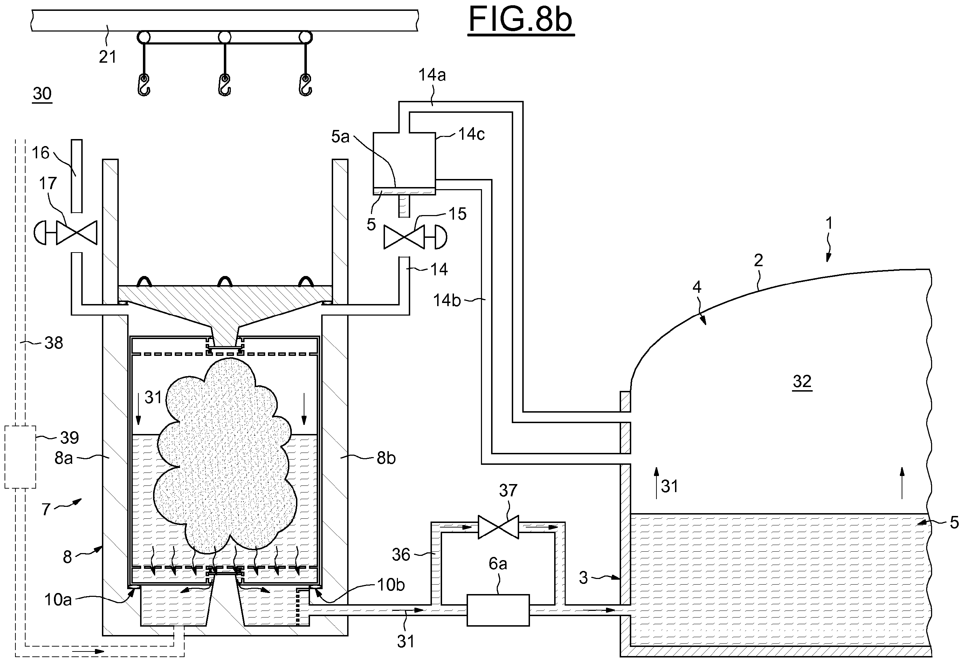

Optionnellement, la figure 8b illustre schématiquement une étape de vidange aérobie mise en œuvre par injection d'air en l'absence du pompage du digestat liquide 5 par le biais de la pompe 6.

La figure 8b illustre un circuit 38 permettant d'injecter de l'air au moyen d'un compresseur 39 dans la partie inférieure de la cuve 8 du digesteur 7. Sur la figure 8b est également représenté un circuit de tuyauterie 36 connectée sur la tuyauterie 6b en amont de la pompe 6a.

Ainsi, au cours de l'étape de vidange aérobie, les vannes 15 et 17 sont fermées tandis que la vanne 37 du circuit de tuyauterie 36 est ouverte. La pompe 6a n'est pas utilisée au cours de cette étape de vidange. Le circuit 38 injecte de l'air au moyen du compresseur 39 de manière à ce que l'air s'accumule dans la partie supérieure de la cuve 8 du digesteur 7 chassant ainsi progressivement par pression le digestat liquide par le bas de la cuve 8. Le digestat liquide 5 est ainsi transféré vers l'unité de digestion principale 1 par le circuit de tuyauterie 36 en contournant la pompe 6a.

L'étape de vidange aérobie est ainsi réalisée de manière alternative sans mettre en œuvre la pompe 6a.

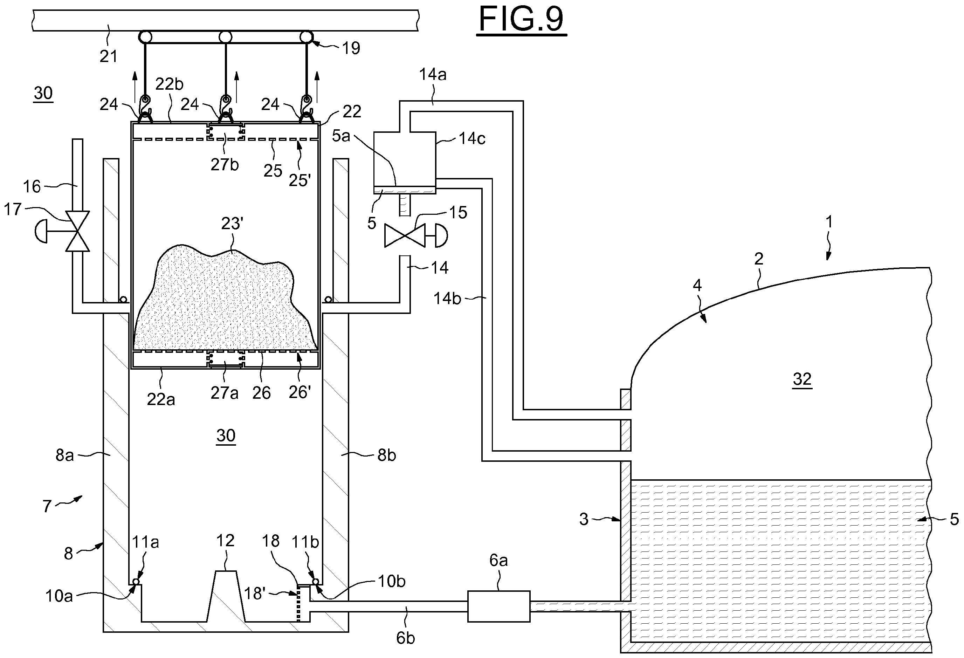

La figure 9 décrit l'étape d'évacuation du container 22 contenant la biomasse solide digérée résiduelle 23', une fois la digestion terminée, par le système d'accrochage 19. En particulier, le toit amovible 24 est préalablement enlevé de manière à ce que le système d'accrochage 19 puisse être actionné à travers l'orifice d'entrée 9 de la cuve 8. De cette façon, le système d'accrochage 19 permet de saisir le container 22 contenant la biomasse solide digérée résiduelle 23', une fois la digestion terminée, de manière à l'évacuer soit vers une zone de stockage, une zone de traitement ou un engin de transport.

Dans le mode de réalisation illustré sur la figure 9, le système d'accrochage 19 saisit le container 22 contenant la biomasse solide digérée résiduelle 23' dans la cuve 8 du digesteur 7.

Grâce à l'étape de vidange aérobie précédente, l'étape de récupération du container 22 contenant la biomasse solide digérée résiduelle 23' après méthanisation, correspondant au digestat solide, peut se dérouler en toute sécurité en minimisant les risques de rejet de biogaz dans l'atmosphère ainsi que les risques de mise en contact avec le biogaz des personnes, du système d'accrochage et des équipements situés en dehors de la cuve 8 de digestion.

Ainsi l'orifice d'entrée 9 de la cuve 8 représente à la fois une zone d'admission du container 22 contenant la biomasse solide 23 avant méthanisation ainsi qu'une zone d'évacuation de ce même container 22 contenant de la biomasse solide digérée résiduelle 23' après méthanisation, appelée digestat solide

En d'autres termes, l'orifice d'entrée 9 de la cuve 8 constitue un espace d'introduction du container contenant la biomasse solide et d'évacuation du container contenant de la biomasse solide digérée résiduelle.

La figure 10 représente plus en détails le container 22.

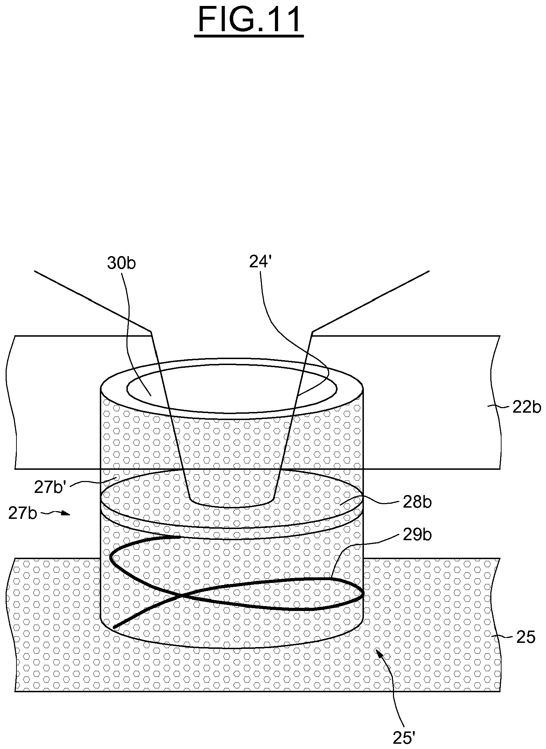

La figure 11 représente plus en détails le système d'ouverture et de fermeture 27b du container 22.

Conformément à cette figure, le système d'ouverture et de fermeture 27b correspond à un cylindre perforé par l'intermédiaire d'une pluralité d'orifices 27b' ayant des tailles identiques ou différentes. De préférence, le système d'ouverture et de fermeture 27b est un cylindre en tôle perforé.

La base inférieure du système d'ouverture et de fermeture 27b est soudée à l'élément de séparation perforé supérieur 25 tandis que la base supérieure du système d'ouverture et de fermeture 27b est soudée à la surface supérieure 22b du container. Un joint d'étanchéité entre la base supérieure du système d'ouverture et de fermeture 27b et la surface supérieure 22b du container 22 est préférentiellement installé.

La figure 11 illustre schématiquement l'action exercée par l'élément en saillie 24' du toit amovible 24 sur l'élément d'obstruction 28b. Conformément à cette figure, l'élément en saillie va exercer une pression sur l'élément d'obstruction 28b afin de compresser le ressort 29b de manière à légèrement pénétrer dans le trou 30b du container 22. Ainsi l'élément d'obstruction 28b est susceptible de se mouvoir horizontalement en montant et en descendant sous la pression exercée par l'élément en saillie 24'.

Au cours de la percolation, le digestat liquide 5, issu des orifices 25', va circuler à travers les orifices 27b' du système d'ouverture et de fermeture 27b.

Le système d'ouverture et de fermeture 27a fonctionne de manière similaire au système d'ouverture et de fermeture 27b sous l'action exercée par l'élément en saillie 12.

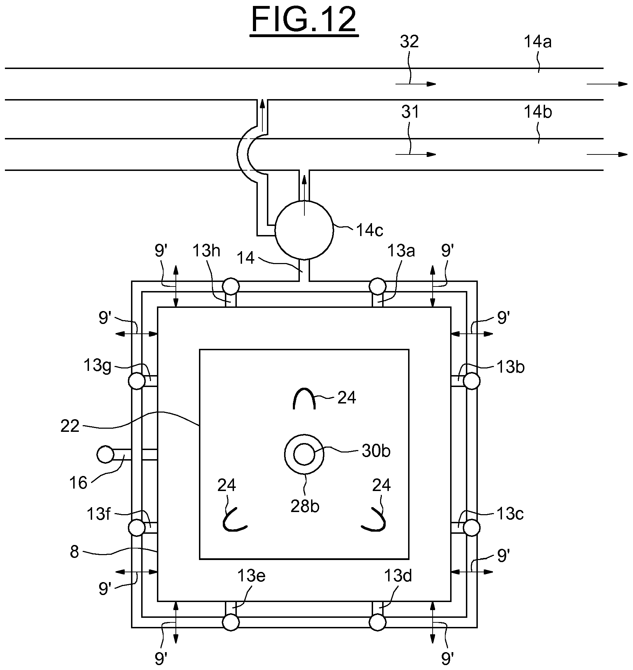

Sur la figure 12 est représentée de manière schématique une vue de dessus de la cuve 8 du digesteur, selon l'invention, ainsi que le container 22 incorporé à l'intérieur de la cuve 8.

En particulier, le container 22 se trouve à l'intérieur de l'orifice d'entrée 9 de la cuve 8 ce qui constitue une zone d'introduction du container contenant de la biomasse solide 23 et un zone d'évacuation du container contenant de la biomasse solide digérée résiduelle 23' après réaction de méthanisation.

Le digesteur 7 présente également une zone 9' d'évacuation du biogaz 32 et d'introduction et d'évacuation d'air.

La partie supérieure de la cuve 8, dans son espace d'évacuation du biogaz 9', est dotée de plusieurs tuyauteries de débordement 13a, 13b, 13c et 13d ... qui sont reliées à une tuyauterie 14 comportant un pot de séparation 14c connecté à deux circuits distincts 14a et 14b qui sont reliés à l'unité de digestion principale 1 (non représentée sur la figure 10).

Le circuit 14a constitue une conduite générale acheminant principalement du biogaz et le circuit 14b constitue une conduite générale acheminant principalement du digestat liquide.

Comme indiqué précédemment, au cours de l'étape de digestion, un mélange de biogaz 32 et de digestat liquide 5 est extrait à travers les tuyauteries de débordement 13a, 13b, 13c, 13d... pour être acheminé dans la tuyauterie 14.

Le digestat liquide 5 se retrouve ensuite acheminé principalement dans le circuit 14b vers la réserve en digestat liquide 5 d'une unité de digestion principale 1 et le biogaz 32 se retrouve acheminé principalement dans le circuit 14a vers l'espace de stockage de gaz 4 de l'unité de digestion principale 1.

La partie supérieure de la cuve 8, dans son espace d'évacuation du biogaz 9', est aussi dotée d'une tuyauterie 16, permettant l'introduction ou l'évacuation de l'air durant certaines phases de chargement aérobie du digestat liquide ou de déchargement aérobie du digestat liquide 5.

Sur la figure 12, le container 22 comprend des anses 24 au niveau de la surface supérieure 22b qui sont configurées de manière à pouvoir s'accrocher au système d'accrochage 19. Les anses 24 du container seront avantageusement au nombre de trois. La surface supérieure 22b comprend également un trou 30b qui est obturé au moyen d'un élément d'obstruction 28b.

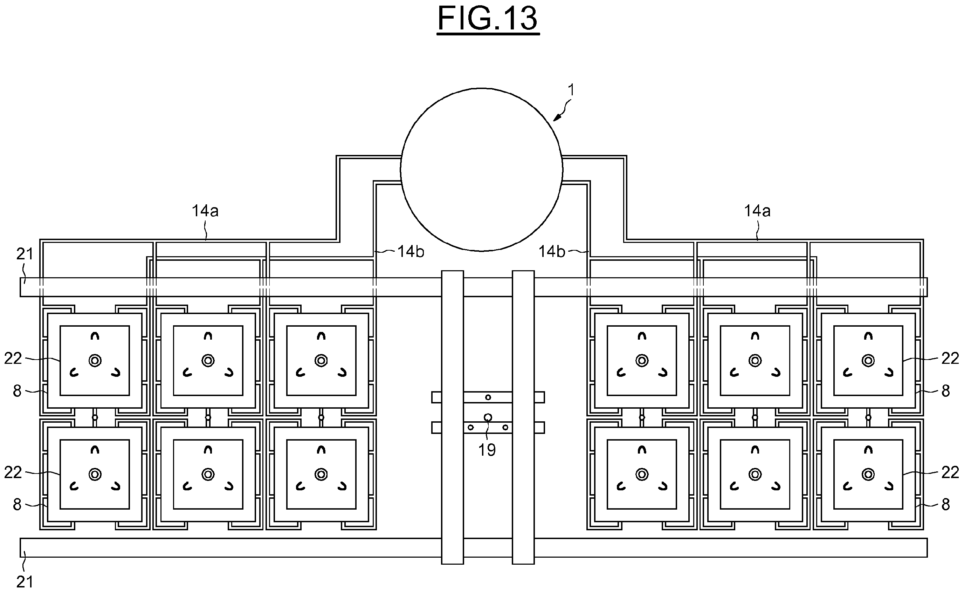

La figure 13 représente schématiquement une vue de dessus d'un système ou d'une installation de production de biogaz comprenant l'unité de digestion principale 1 susceptible d'être alimentée par débordement en digestat liquide, via un circuit d'acheminement 14b, par une pluralité de digesteurs 7, chacun étant alimenté par le haut par un container 22 contenant de la biomasse solide 23 par le biais d'un système d'accrochage 19 monté sur un pont roulant 21.

L'unité de digestion principale 1 est également alimentée en biogaz par le biais d'un circuit d'acheminement 14a suite à la production de biogaz dans chaque digesteur 7.

L'unité de digestion principale 1 n'est pas, ou très faiblement, alimentée en biomasse solide 23 au cours du procédé de méthanisation mais constitue avantageusement une réserve en digestat liquide 5 et un espace de stockage en biogaz 32.

La réserve en digestat liquide 5 sert donc à alimenter les digesteurs 7 et l'espace de stockage 4 en biogaz 32 sert à récupérer et stocker le biogaz 32 issu des différents digesteurs 7.

Au cours du procédé de méthanisation considéré dans son ensemble, le système d'accrochage 19 circule au dessus des différents digesteurs 7 afin de les alimenter par le haut avec des containers 22 contenant de la biomasse solide 23.

De la même façon, le système d'accrochage 19 circule au dessus des différents digesteurs 7 afin de récupérer par le haut les containers 22 contenant les biomasses solides digérées résiduelles 23', ou digestats solides, en fin de cycle de méthanisation.

Bien que la figure 13 représente une pluralité de digesteurs 7 ayant une taille identique, le système de production de biogaz selon l'invention peut comprendre une pluralité de digesteurs ayant des tailles différentes entre eux.

De plus, bien que la figure 13 représente une unité de digestion principale composée d'un seul ouvrage, cette dernière peut être constituée de deux ouvrages distincts, l'un destiné à contenir principalement du digestat liquide, l'autre destiné à contenir principalement du biogaz.

## Revendications

1. Système de production de biogaz (32) comportant :
- au moins une unité de digestion principale (1), constitué d'un ou plusieurs ouvrages, apte à contenir du biogaz (32) et du digestat liquide (5),
- au moins un digesteur de méthanisation (7), destiné à la production de biogaz (32), comportant une cuve (8), apte à contenir un container (22) contenant de la biomasse solide (23), ledit container étant apte à se remplir de digestat liquide (5), et ledit digesteur (7) comportant : au moins une zone (9) d'introduction du container (22) contenant de la biomasse solide (23) et d'évacuation du container (22) contenant de la biomasse solide digérée résiduelle (23'), ladite zone (9) sur laquelle un toit (24) est apte à s'ouvrir et à se refermer, et au moins une zone (9') d'évacuation du biogaz (32),
- ledit digesteur de méthanisation (7) étant relié(s) par le biais d'un circuit d'alimentation (6b) en digestat liquide (5) et d'un circuit d'évacuation (14) de biogaz (32) et de digestat liquide (5) à ladite unité de digestion principale (1).

2. Système de production de biogaz (32) selon la revendication 1, **caractérisé en ce que** ledit digesteur de méthanisation (7) est surmonté d'un système d'accrochage (19) apte à permettre l'introduction du container (22) contenant la biomasse solide (23) à l'intérieur de la cuve (8) et l'évacuation du container (22) contenant de la biomasse solide digérée résiduelle (23').

3. Système de production de biogaz (32) selon la revendication 2, **caractérisé en ce que** le système d'accrochage (19) comporte trois crochets.

4. Système de production de biogaz (32) selon la revendication 2 ou 3, **caractérisé en ce que** le système d'accrochage (19) est apte à ouvrir et refermer le toit (24) dudit digesteur (7).

5. Système de production de biogaz (32) selon l'une quelconque des revendications 1 à 4, **caractérisé en ce que** ledit digesteur (7) comprend au moins un élément de séparation perforé inférieur (18), situé au niveau de la partie inférieure de la cuve (8), apte à séparer le digestat liquide (5) de la biomasse solide (23).

6. Système de production de biogaz (32) selon l'une quelconque des revendications 1 à 5, **caractérisé en ce que** ledit digesteur comprend une ouverture (16) munie d'une vanne (17), apte à obturer ou permettre le passage de l'air (24) entre l'intérieur et l'extérieur de la cuve (8) du digesteur (7) et une vanne (15) située sur un circuit d'acheminement (14), apte à obturer ou permettre la circulation de biogaz (25) et/ou de digestat liquide (5) entre le digesteur (7) et une unité de digestion principale (1).

7. Système de production de biogaz (32) selon l'une quelconque des revendications 1 à 6, **caractérisé en ce que** ledit digesteur comporte un bouchon (33) positionné sur le toit (24) de la cuve (8).

8. Système de production de biogaz (32) selon la revendication 7, **caractérisé en ce que** le bouchon (33) positionné sur le toit (24) de la cuve (8) est un bouchon liquide.

9. Système de production de biogaz (32) selon l'une quelconque des revendications précédentes, **caractérisé en ce que** l'unité de digestion principale (1) est ou sont constituées par deux ouvrages distincts, l'un destiné à contenir principalement du digestat liquide (5), l'autre destiné à contenir principalement du biogaz (32).

10. Système de production de biogaz (32) selon l'une quelconque des revendications précédentes, **caractérisé en ce que** le circuit d'évacuation (14) comprend un circuit (14a) apte à acheminer du biogaz (32) et un circuit (14b) apte à acheminer du digestat liquide (5).

11. Système de production de biogaz (32) selon la revendication 10, **caractérisé en ce que** le circuit d'évacuation (14) comprend un pot de séparation (14c) apte à séparer le biogaz (32) et le digestat liquide (5)

12. Procédé de production de biogaz (32) comprenant au moins les étapes successives suivantes :
- une étape d'introduction d'un container (22) contenant de la biomasse solide (23), mise en oeuvre par au moins un système d'accrochage (19), à travers une zone (9) d'admission et d'évacuation située au niveau de la partie supérieure d'une cuve (8) du digesteur (7), telle que définie selon la revendication 1, et sur laquelle un toit (24) est apte à s'ouvrir et à se refermer,
- une étape de remplissage de la cuve (8) dudit digesteur (7) contenant le container (22) avec un digestat liquide (5) pour immerger la biomasse solide (23) et éventuellement mettre en suspension une partie de ladite biomasse solide (23) à l'intérieur du container (22),
- une étape de digestion, constituée d'une percolation du digestat liquide (5) à travers la biomasse solide (23) contenue dans le container (22) et d'une diffusion du digestat liquide (5) dans la biomasse solide (23) dans des conditions anaérobies pour générer puis récupérer le biogaz (32) à travers une zone d'évacuation (9'), située dans la partie supérieure dudit digesteur (7), et
- une étape de vidange aérobie du digestat liquide (5) de la cuve (8) du digesteur (7), et
- une étape d'évacuation du container (22) contenant de la biomasse solide digérée résiduelle (23'), mise en œuvre par au moins un système d'accrochage (19), à travers la zone (9) du digesteur (7).

13. Procédé de production de biogaz (32) selon la revendication 12, **caractérisé en ce qu'**il comprend en outre entre l'étape de digestion et l'étape de vidange aérobie du digestat liquide (5) au moins les étapes suivantes :
- une étape de vidange anaérobie du container (22) du digestat liquide (5) de la cuve (8) du digesteur (7), et
- une étape de re-remplissage de la cuve (8) du digesteur (7) avec un digestat liquide (5) après vidange anaérobie

14. Procédé de production de biogaz (32) selon les revendications 12 et 13, **caractérisé en ce qu'**il comprend en outre, entre l'étape de re-remplissage de la cuve (8) du digesteur (7) avec un digestat liquide (5) et l'étape de vidange aérobie de la biomasse liquide, au moins une étape de digestion, constituée d'une percolation du digestat liquide (5) à travers la biomasse solide (23) contenue dans le container (22) et d'une diffusion du digestat liquide (5) dans la biomasse solide (23) pour générer puis récupérer le biogaz (32) à travers la zone (9')

15. Procédé de production de biogaz (32) selon la revendication 14, **caractérisé en ce qu'**il comprend en outre, pendant l'étape de digestion, une étape d'injection de biogaz dans la partie inférieure de la cuve (8) du digesteur (7) à l'aide d'un compresseur de biogaz (34).

16. Container apte à être introduit dans la cuve (8) du digesteur (7) tel que défini selon la revendication 1, **caractérisé en ce qu'**il comprend un élément de séparation perforé supérieur (25) et un élément de séparation perforé inférieur (26), aptes à laisser circuler du digestat liquide (5) et à retenir la biomasse solide non digérée (23) ou digérée (23') et un système d'ouverture et de fermeture (27a), fixé sur la surface inférieure (22a) du container (22), ledit système (27a) étant apte à s'ouvrir au contact avec le fond de la cuve (8) du digesteur (7).

17. Container selon la revendication 16, **caractérisé en ce qu'**il comprend un système d'ouverture et de fermeture (27b), fixé sur la surface supérieure (22b) du container (22), ledit système (27b) étant apte à s'ouvrir au contact avec le toit (24) du digesteur (7).

## Patentansprüche

1. Biogas-Erzeugungssystem (32), das aufweist:
- mindestens eine aus einem oder mehreren Bauteilen bestehende Hauptvergärungsanlage (1), die Biogas (32) und flüssiges Gärgut (5) enthalten kann,
- mindestens einen zur Erzeugung von Biogas (32) bestimmten Vergärungs-Fermenter (7), der einen Behälter (8) aufweist, der einen feste Biomasse (23) enthaltenden Container (22) enthalten kann, wobei der Container sich mit flüssigem Gärgut (5) füllen kann, und der Fermenter (7) aufweist: mindestens einen Bereich (9) zur Einführung des feste Biomasse (23) enthaltenden Containers (22) und zur Beseitigung des restliche vergärte feste Biomasse (23') enthaltenden Containers (22), den Bereich (9), auf dem ein Dach (24) sich öffnen und schließen kann, und mindestens einen Bereich (9') zum Beseitigen des Biogases (32),
- wobei der Vergärungs-Fermenter (7) über einen Versorgungskreislauf (6b) mit flüssigem Gärgut (5) und über einen Beseitigungskreislauf (14) von Biogas (32) und flüssigem Gärgut (5) mit der Hauptvergärungsanlage (1) verbunden ist.

2. Biogaserzeugungssystem (32) nach Anspruch 1, **dadurch gekennzeichnet, dass** über dem Vergärungs-Fermenter (7) ein Befestigungssystem (19) angeordnet ist, das die Einführung des die feste Biomasse (23) enthaltenden Containers (22) ins Innere des Behälters (8) und die Beseitigung des die restliche vergärte feste Biomasse (23') enthaltenden Containers (22) erlauben kann.

3. Biogaserzeugungssystem (32) nach Anspruch 2, **dadurch gekennzeichnet, dass** das Befestigungssystem (19) drei Haken aufweist.

4. Biogaserzeugungssystem (32) nach Anspruch 2 oder 3, **dadurch gekennzeichnet, dass** das Befestigungssystem (19) das Dach (24) des Fermenters (7) öffnen und schließen kann.

5. Biogaserzeugungssystem (32) nach einem der Ansprüche 1 bis 4, **dadurch gekennzeichnet, dass** der Fermenter (7) mindestens ein im Bereich des unteren Teils des Behälters (8) befindliches unteres gelochtes Trennelement (18) enthält, das das flüssige Gärgut (5) von der festen Biomasse (23) trennen kann.

6. Biogaserzeugungssystem (32) nach einem der Ansprüche 1 bis 5, **dadurch gekennzeichnet, dass** der Fermenter eine Öffnung (16), die mit einem Ventil (17) versehen ist, das den Luftdurchgang (24) zwischen dem Inneren und Äußeren des Behälters (8) des Fermenters (7) verschließen oder erlauben kann, und ein auf einem Beförderungskreislauf (14) befindliches Ventil (15) enthält, das den Kreislauf von Biogas (25) und/oder von flüssigem Gärgut (5) zwischen dem Fermenter (7) und einer Hauptvergärungsanlage (1) verschließen oder erlauben kann.

7. Biogaserzeugungssystem (32) nach einem der Ansprüche 1 bis 6, **dadurch gekennzeichnet, dass** der Fermenter einen Verschluss (33) aufweist, der auf dem Dach (24) des Behälters (8) positioniert ist.

8. Biogaserzeugungssystem (32) nach Anspruch 7, **dadurch gekennzeichnet, dass** der auf dem Dach (24) des Behälters (8) positionierte Verschluss (33) ein flüssiger Verschluss ist.

9. Biogaserzeugungssystem (32) nach einem der vorhergehenden Ansprüche, **dadurch gekennzeichnet, dass** die Hauptvergärungsanlage (1) aus zwei unterschiedlichen Bauteilen besteht oder bestehen, von denen eines dazu bestimmt ist, hauptsächlich flüssiges Gärgut (5) zu enthalten, und das andere dazu bestimmt ist, hauptsächlich Biogas (32) zu enthalten.

10. Biogaserzeugungssystem (32) nach einem der vorhergehenden Ansprüche, **dadurch gekennzeichnet, dass** der Beseitigungskreislauf (14) einen Kreislauf (14a), der Biogas (32) befördern kann, und einen Kreislauf (14b) enthält, der flüssiges Gärgut (5) befördern kann.

11. Biogaserzeugungssystem (32) nach Anspruch 10, **dadurch gekennzeichnet, dass** der Beseitigungskreislauf (14) einen Trenntopf (14c) enthält, der das Biogas (32) und das flüssige Gärgut (5) trennen kann.

12. Verfahren zur Erzeugung von Biogas (32), das mindestens die folgenden aufeinanderfolgenden Schritte enthält:
- einen Schritt der Einführung eines feste Biomasse (23) enthaltenden Containers (22), der von mindestens einem Befestigungssystem (19) durchgeführt wird, durch einen Einlass- und Beseitigungsbereich (9) hindurch, der sich im Bereich des oberen Teils eines Behälters (8) des Fermenters (7) befindet, wie gemäß Anspruch 1 definiert, und auf dem ein Dach (24) sich öffnen und schließen kann,
- einen Schritt des Füllens des den Container (22) enthaltenden Behälters (8) des Fermenters (7) mit einem flüssigen Gärgut (5), um die feste Biomasse (23) einzutauchen und ggf. einen Teil der festen Biomasse (23) im Inneren des Containers (22) zu suspendieren,
- einen Schritt der Gärung, bestehend aus einer Versickerung des flüssigen Gärguts (5) durch die im Container (22) enthaltene feste Biomasse (23) hindurch und einer Diffusion des flüssigen Gärguts (5) in die feste Biomasse (23) unter anaeroben Bedingungen, um das Biogas (32) zu erzeugen und über einen Beseitigungsbereich (9') wiederzugewinnen, der sich im oberen Teil des Fermenters (7) befindet, und
- einen Schritt der aeroben Entleerung des flüssigen Gärguts (5) aus dem Behälter (8) des Fermenters (7), und
- einen Schritt der Beseitigung des restliche vergärte feste Biomasse (23') enthaltenden Containers (22), der von mindestens einem Befestigungssystem (19) durch den Bereich (9) des Fermenters (7) hindurch durchgeführt wird.

13. Verfahren zur Erzeugung von Biogas (32) nach Anspruch 12, **dadurch gekennzeichnet, dass** es außerdem zwischen dem Gärungsschritt und dem Schritt der aeroben Entleerung des flüssigen Gärguts (5) mindestens die folgenden Schritte enthält:
- einen Schritt der anaeroben Entleerung aus dem Container (22) des flüssigen Gärguts (5) des Behälters (8) des Fermenters (7), und
- einen Schritt des Wiederbefüllens des Behälters (8) des Fermenters (7) mit einem flüssigen Gärgut (5) nach der anaeroben Entleerung.

14. Verfahren zur Erzeugung von Biogas (32) nach den Ansprüchen 12 und 13, **dadurch gekennzeichnet, dass** es außerdem zwischen dem Schritt des Wiederbefüllens des Behälters (8) des Fermenters (7) mit einem flüssigen Gärgut (5) und dem Schritt des aeroben Entleerens der flüssigen Biomasse mindestens einen Gärungsschritt enthält, der aus einer Versickerung des flüssigen Gärguts (5) durch die im Container (22) enthaltene feste Biomasse (23) hindurch und einer Diffusion des flüssigen Gärguts (5) in die feste Biomasse (23) besteht, um das Biogas (32) zu erzeugen und dann durch den Bereich (9') hindurch wiederzugewinnen.

15. Verfahren zur Erzeugung von Biogas (32) nach Anspruch 14, **dadurch gekennzeichnet, dass** es außerdem während des Gärungsschritts einen Schritt des Injizierens von Biogas in den unteren Teil des Behälters (8) des Fermenters (7) mit Hilfe eines Biogasverdichters (34) enthält.

16. Container, der in den Behälter (8) des Fermenters (7) wie gemäß Anspruch 1 definiert eingeführt werden kann, **dadurch gekennzeichnet, dass** er ein oberes gelochtes Trennelement (25) und ein unteres gelochtes Trennelement (26), die flüssiges Gärgut (5) zirkulieren lassen und die nicht vergärte (23) oder vergärte (23') feste Biomasse zurückhalten können, und ein Öffnungs- und Schließsystem (27a) enthält, das an der unteren Fläche (22a) des Containers (22) befestigt ist, wobei das System (27a) sich in Kontakt mit dem Boden des Behälters (8) des Fermenters (7) öffnen kann.

17. Container nach Anspruch 16, **dadurch gekennzeichnet, dass** er ein Öffnungs- und Schließsystem (27b) enthält, das an der Oberfläche (22b) des Containers (22) befestigt ist, wobei das System (27b) sich in Kontakt mit dem Dach (24) des Fermenters (7) öffnen kann.

## Claims

1. A system for production of biogas (32) comprising:
- at least one main digestion unit (1), constituted by one or a plurality of elements which can contain biogas (32) and liquid digestate (5);
- at least one methanization digester (7), which is designed for the production of biogas (32), comprising a tank (8) which can contain a container (22) containing solid biomass (23), said container being able to be filled with liquid digestate (5) and said digester (7) comprising: at least one area (9) for introduction of the container (22) containing solid biomass (23) and for discharge of the container (22) containing residual digested solid biomass (23'), said area (9) on which a roof (24) can be opened and closed, and at least one area (9') for discharge of the biogas (32);
- said methanization digester (7) being connected by means of a circuit (6b) for supply of liquid digestate (5) and a circuit (14) for discharge of biogas (32) and liquid digestate (5) to and from said main digestion unit (1).

2. The system for production of biogas (32) as claimed in claim 1, **characterized in that** said methanization digester (7) is surmounted by a coupling system (19) which can permit the introduction of the container (22) containing the solid biomass (23) into the interior of the tank (8), and discharge of the container (22 containing residual digested solid biomass (23').

3. The system for production of biogas (32) as claimed in claim 2, **characterized in that** the coupling system (19) comprises three hooks.

4. The system for production of biogas (32) as claimed in claim 2 or 3, **characterized in that** the coupling system (19) can open and close the roof (24) of said digester (7).

5. The system for production of biogas (32) as claimed in any one of claims 1 to 4, **characterized in that** said digester (7) comprises at least one lower perforated separation element (18), situated in the lower part of the tank (8), which can separate the liquid digestate (5) from the solid biomass (23).

6. The system for production of biogas (32) as claimed in any one of claims 1 to 5, **characterized in that** said digester comprises an opening (16) provided with a valve (17) which can shut off or permit the passage of the air (24) between the interior and the exterior of the tank (8) of the digester (7), and a valve (15) situated on a conveying circuit (14), which can shut off or permit the circulation of biogas (25) and/or liquid digestate (5), between the digester (7) and a main digestion unit (1).

7. The system for production of biogas (32) as claimed in any one of the claims 1 to 6, **characterized in that** said digester comprises a stopper (33) positioned on the roof (24) of the tank (8).

8. The system for production of biogas (32) as claimed in claim 7, **characterized in that** the stopper (33) positioned on the roof (24) of the tank (8) is a liquid stopper.

9. The system for production of biogas (32) as claimed in any one of the preceding claims, **characterized in that** the main digestion unit (1) is constituted by two distinct elements, one designed to contain mainly liquid digestate (5), the other designed to contain mainly biogas (32).

10. The system for production of biogas (32) as claimed in any one of the preceding claims, **characterized in that** the discharge circuit (14) comprises a circuit (14a) which can convey biogas (32), and a circuit (14b) which can convey liquid digestate (5).

11. The system for production of biogas (32) as claimed in claim 10, **characterized in that** the discharge circuit (14) comprises a separation chamber (14c) which can separate the biogas (32) and the liquid digestate (5).

12. A process for production of biogas (32) comprising at least the following successive steps:
- a step of introduction of a container (22) containing solid biomass (23), implemented by at least one coupling system (19), through an area (9) for intake and discharge situated in the upper part of a tank (8) of the digester (7), as defined in claim 1, and on which a roof (24) can open and close;
- a step of filling the tank (8) of said digester (7) containing the container (22) with a liquid digestate (5), in order to immerse the solid biomass (23) and optionally put part of said solid biomass (23) into suspension in the interior of the container (22);
- a step of digestion constituted by percolation of the liquid digestate (5) through the solid biomass (23) contained in the container (22), and diffusion of the liquid digestate (5) in the solid biomass (23) in anaerobic conditions, in order to generate the biogas (32), then recuperate it through a discharge area (9') situated in the upper part of said digester (7); and
- a step of aerobic emptying of the liquid digestate (5) from the tank (8) of the digester (7); and
- a step of discharge of the container (22) containing residual digested solid biomass (23'), implemented by at least one coupling system (19), through the area (9) of the digester (7).

13. The process for production of biogas (32) as claimed in claim 12, **characterized in that** it additionally comprises at least the following steps between the step of digestion and the step of aerobic emptying of the liquid digestate (5):
- a step of anaerobic emptying from the container (22) of the liquid digestate (5) of the tank (8) of the digester (7); and
- a step of refilling of the tank (8) of the digester (7) with liquid digestate (5) after anaerobic emptying.

14. The process for production of biogas (32) as claimed in claims 12 and 13, **characterized in that** it additionally comprises, between the step of re-filling of the tank (8) of the digester (7) with a liquid digestate (5) and the step of aerobic emptying of the liquid biomass, at least one step of digestion, constituted by percolation of the liquid digestate (5) through the solid biomass (23) contained in the container (22), and diffusion of the liquid digestate (5) in the solid biomass (23), in order to generate then recuperate the biogas (32) through the area (9').

15. The process for production of biogas (32) as claimed in claim 14, **characterized in that** it additionally comprises, during the digestion step, a step of injection of biogas into the lower part of the tank (8) of the digester (7) by means of a biogas compressor (34).

16. A container which can be introduced into the tank (8) of the digester (7) as defined in claim 1, **characterized in that** it comprises an upper perforated separation element (25) and a lower perforated separation element (26), which can allow liquid digestate (5) to circulate, and can retain the undigested (23) or digested (23') solid biomass, and a system (27a) for opening and closure, secured on the lower surface (22a) of the container (22), said system (27a) being able to open in contact with the base of the tank (8) of the digester (7).

17. The container as claimed in claim 16, **characterized in that** it comprises a system (27b) for opening and closure, secured on the upper surface (22b) of the container (22), said system (27b) being able to open in contact with the roof (24) of the digester (7).
